(19) Europäisches Patentamt
European Patent Office
Office européen des brevets

(11) Publication number: **0 098 942**
**B1**

(12) **EUROPEAN PATENT SPECIFICATION**

(45) Date of publication of patent specification: **12.03.86**

(51) Int. Cl.[4]: **C 07 D 233/60,** A 61 K 31/41, A 61 K 31/415

(21) Application number: **83105030.7**

(22) Date of filing: **20.05.83**

(54) **Optically active imidazolylpropanol compounds, and their production and use.**

(30) Priority: **24.05.82 JP 88530/82**

(43) Date of publication of application:
**25.01.84 Bulletin 84/04**

(45) Publication of the grant of the patent:
**12.03.86 Bulletin 86/11**

(84) Designated Contracting States:
**AT BE CH DE FR GB IT LI NL SE**

(56) References cited:
**EP-A-0 001 654**
**EP-A-0 023 103**
**EP-A-0 054 974**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Saji, Ikutaro**
**8-Q-401, Aobaokaminami**
**Suita Osaka (JP)**
Inventor: **Sato, Renzo**
**14-7, Mefu 2-chome**
**Takarazuka Hyogo (JP)**
Inventor: **Aono, Shunji**
**15-33-303, Minamisakurazuka 3-chome**
**Toyonaka Osaka (JP)**
Inventor: **Ichise, Katsuaki**
**2-30-310, Nagaoka 2-chome**
**Nagaokakyo Kyoto (JP)**
Inventor: **Okuda, Takao**
**10-3-337, Sonehigashi-machi 2-chome**
**Toyonaka Osaka (JP)**
Inventor: **Atsumi, Toshio**
**3-45, Seiwadainishi 2-chome**
**Kawanishi Hyogo (JP)**
Inventor: **Hanma, Noritaka**
**140-15, Hikishoharadera-machi**
**Sakai Osaka (JP)**
Inventor: **Motoike, Yasuo**
**9-17, Sakuragaoka 4-chome**
**Minoo Osaka (JP)**

**EP 0 098 942 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Vossius Vossius Tauchner Heunemann Rauh**
**Siebertstrasse 4 P.O. Box 86 07 67**
**D-8000 München 86 (DE)**

**Description**

The present invention relates to optically active imidazolylpropanol compounds, and their production and use. More particularly, it relates to optically active imidazolylpropanol compounds of the formula:

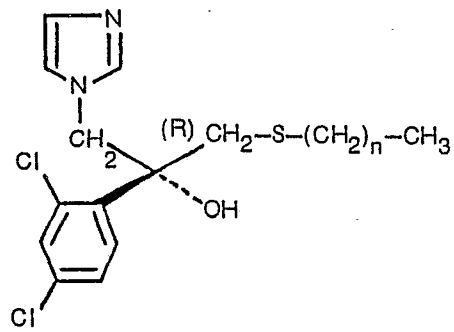

(I)

wherein n is an integer of 3 or 4, and their acid addition salts, and their preparation process, and their antifungal use.

The racemic mixture of an imidazolylpropanol compound corresponding to the formula (I) is known to be useful as an antifungal agent (cf. JP—A—106666/1982). Similar compounds are disclosed in EP—A—23103. In order to provide more active compounds, an extensive study has been made, and it has now been found that the (R)-isomer of the imidazolylpropanol compound (I) is highly active.

On Candida infection in mice, the said (R)-isomer showed a more remarkable effect in decreasing mortality than the corresponding racemate or (S)-isomer. Thus, the (R)-isomer is particularly useful as an antifungal agent.

The optically active imidazolylpropanol compound of the formula (I), i.e. the (R)-isomer, can be prepared according to the following diagrammatic scheme (Method A):

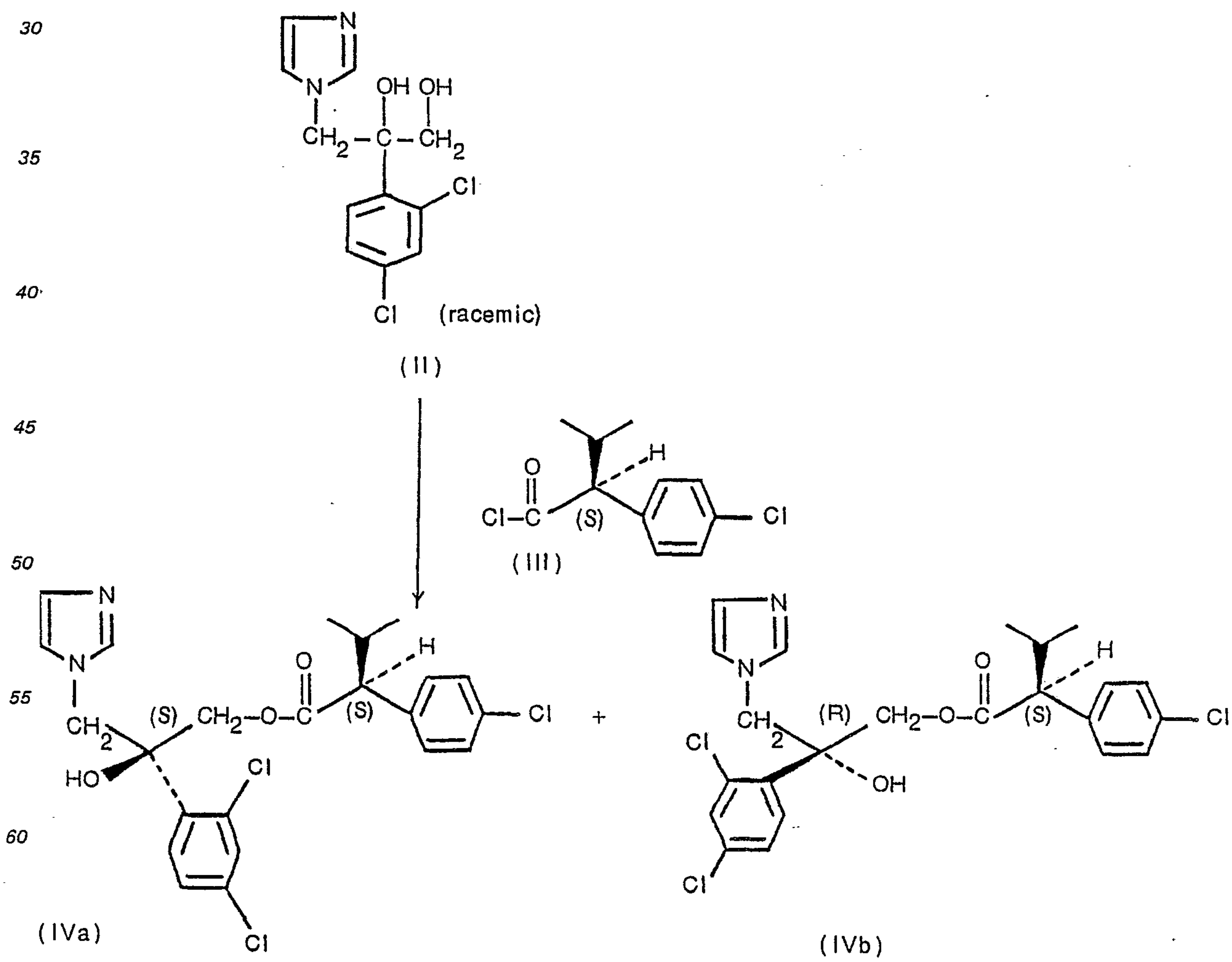

(racemic)

(II)

(III)

(IVa)

(IVb)

(IVb)

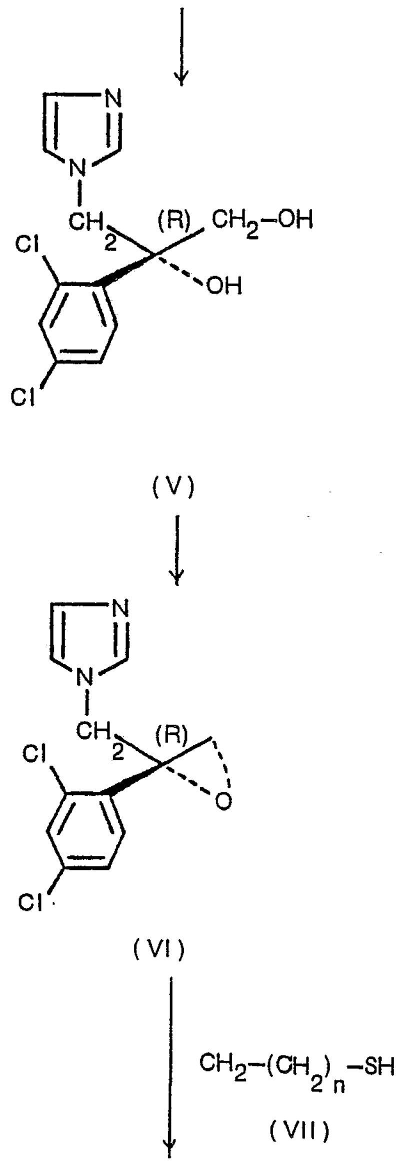


(I)

In Method A, the racemic diol of the formula (II) is first reacted with the optically acitve acid chloride of the formula (III) in the presence of a tertiary amine, usually in an inert solvent to give a 1:1 diastereomeric mixture of the esters (IVa) and (IVb).

The racemic diol (II) is disclosed in JP—A—149274/1982, whereas the optically active acid chloride (III) is described in J. Synthetic Organic Chemistry, Japan, *38*, 1151—1162 (1980). The optically active acid chloride (III) is, however, merely an example, and any other optically active acid chloride may be employed. Suitable examples of such other optically active acid chloride are (+)-α-methoxy-α-trifluoromethylphenylacetyl chloride, L-menthoxyacetyl chloride, etc. These optically active acid chlorides can be represented by the formula: A—Cl wherein A is an optically active acyl group. As the tertiary amine, there are exemplified pyridine, N,N-dimethylaniline, triethylamine, etc. Examples of the inert solvent are ethers (e.g. tetrahydrofuran, dimethoxyethane), halogenated hydrocarbons (e.g. chloroform, dichloromethane), aromatic hydrocarbons (e.g. benzene, toluene), etc. The molar equivalent ratio of the racemic diol (II) and the optically active acid chloride (III) is usually from 1:1 to 1.5:1. The amount of the tertiary amine may be usually from 1 to 1.5 molar equivalents to the optically active acid chloride (III). The reaction temperature is normally from 0 to 30°C.

From the thus obtained 1:1 diastereomeric mixture, the (R)-isomer (IVb) is separated and collected by a per se conventional separation procedure such as silica gel column chromatography or fractional crystallization. Examples of solvents usable on the fractional crystallization are alcohols (e.g. methanol, ethanol, isopropanol), ethers (e.g. diethyl ether, tetrahydrofuran), hydrocarbons (e.g. hexane, benzene, toluene), halogenated hydrocarbons (e.g. 1,2-dichloroethane, dichloromethane), acetone, etc.

Then, the (R)-isomer (IVb) is hydrolyzed with a base in an aqueous medium to give the optically active diol of the formula (V). As the base, there may be used, for instance, an alkali metal hydroxide (e.g. sodium hydroxide, potassium hydroxide). The amount of the alkali metal hydroxide may be from 1 to 5 molar equivalents to the (R)-isomer (IVb). The reaction temperature is normally from 0 to 80°C, preferably from 0 to 30°C. When desired, the reaction medium may contain any organic solvent, of which examples are alcohols (e.g. methanol, ethanol), ethers (e.g. tetrahydrofuran, dioxane), etc.

The optically active diol (V) is then reacted with methanesulfonyl chloride, followed by treatment with an alkali to give the epoxide of the formula (VI). For instance, the optically active diol (V), preferably dissolved in a basic solvent (e.g. pyridine, triethylamine), is reacted with methanesulfonyl chloride at a temperature of 0 to 20°C, preferably of 0 to 5°C, and a solution or suspension of an alkali (e.g. potassium hydroxide, sodium hydroxide, sodium methoxide, sodium ethoxide) in a solvent (e.g. methanol, ethanol, benzene, toluene, tetrahydrofuran, dioxane), preferably a methanol solution of potassium hydroxide, is added thereto at a temperature of from 0 to 10°C, followed by stirring at 10 to 20°C for 1 to 3 hours to give the epoxide (VI).

The optically active epoxide (VI) is then reacted with a thiol compound of the formula (VII) in a metallized form in an inert solvent to give the objective optically active imidazolylpropanol compound (I). The amount of the thiol compound (VII) may be usually from 1 to 3 molar equivalents to the epoxide (VI). As the inert solvent, there may be used an ether (e.g. tetrahydrofuran, dimethoxyethane), dimethylformamide or the like. The reaction temperature is normally from 0 to 100°C, preferably from 0 to 30°C. Prior to the reaction, the thiol compound (VII) is converted into its metallized form, and the conversion can be achieved by reacting the thiol compound (VII) with an equimolar amount of an alkali metal (e.g. metallic potassium, metallic sodium, metallic lithium) or an alkali metal hydride (e.g. sodium hydride) in a liquid medium.

The optically active imidazolylpropanol compound (I) can be also prepared according to the following diagrammatic scheme (Method B):

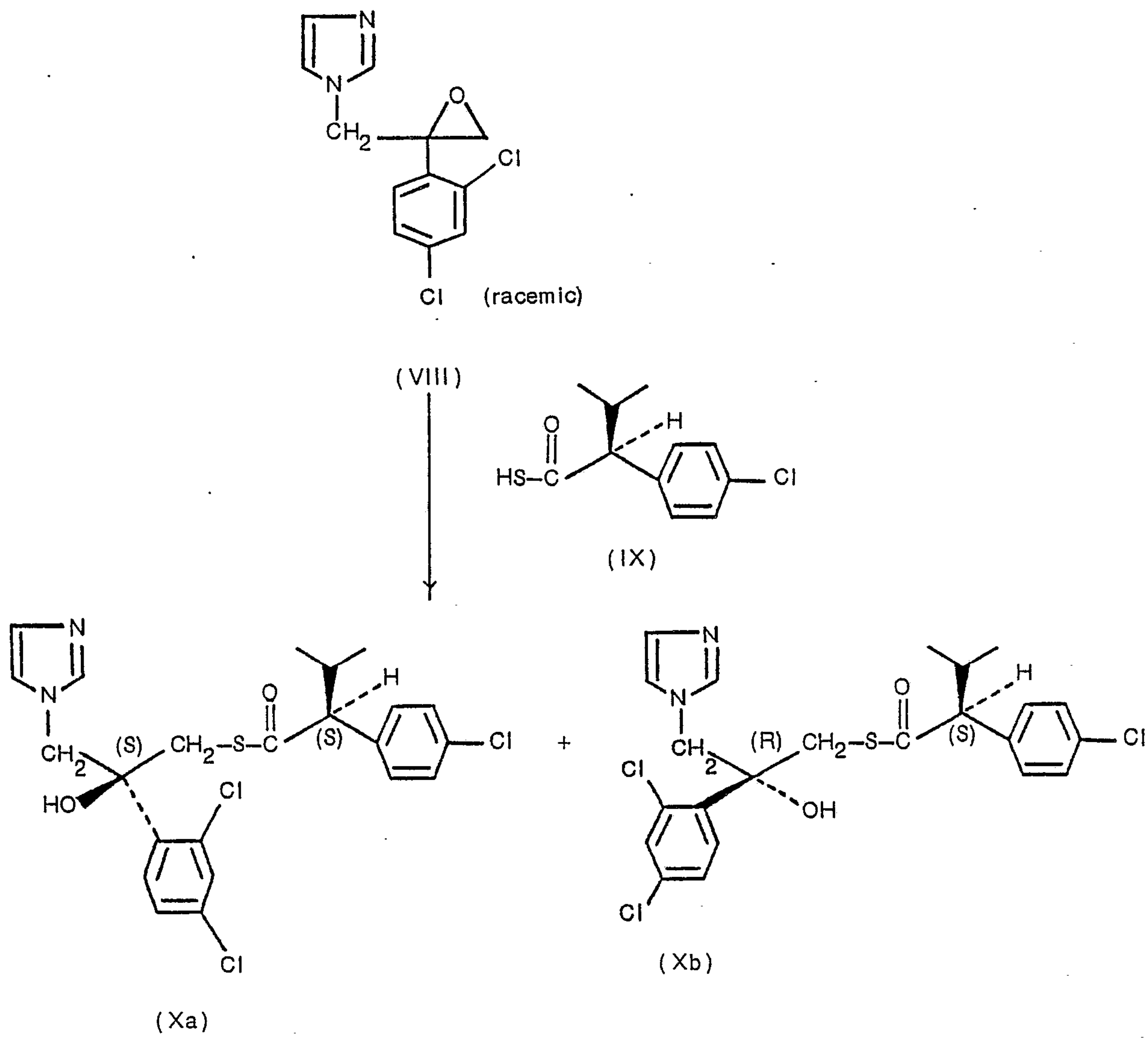

(racemic)

(VIII)

(IX)

(Xa)

(Xb)

(Xb)

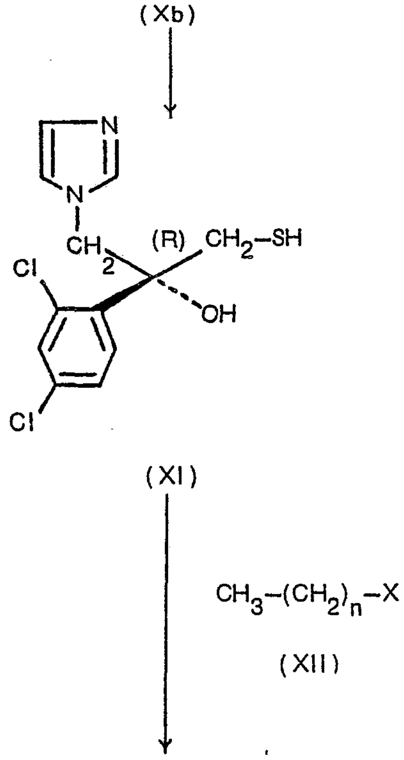


(XI)

(I)

In Method B, the racemic epoxide of the formula (VIII) is first reacted with an optically active thiolocarboxylic acid of the formula (IX) in an inert solvent at a temperature of from about −20 to 80°C to give a 1:1 diastereomeric mixture of the thioloesters (Xa) and (Xb).

The racemic epoxide (VIII) is known (cf. JP—A— Nos. 12372/1981 and 106666/1982). The optically active thiolocarboxylic acid (IX) can be prepared from the corresponding carboxylic acid chloride (III) by reacting the latter with potassium or sodium hydrosulfide in an inert solvent such as a hydrocarbon (e.g. benzene, toluene) or an alcohol (e.g. methanol, ethanol) at a temperature of about −10 to 10°C (cf. Org. Synthesis, Col. Vol. III, p. 116). The optically active thiolocarboxylic acid (IX) is merely a typical example, and other optically active thiolocarboxylic acids such as (+)-α-methoxy-α-trifluoromethylphenylthioloacetic acid and L-menthoxythioloacetic acid may be also used. These optically active thiolocarboxylic acids are representable by the formula: A—SH wherein A is as defined above. Examples of the inert solvent are hydrocarbons (e.g. n-hexane, benzene, xylene), alcohols (e.g. methanol, ethanol, isopropanol), halogenated hydrocarbons (e.g. dichloromethane, chloroform, 1,2-dichloroethane), ketones (e.g. acetone, methyl ethyl ketone), ethers (e.g. diethyl ether, tetrahydrofuran, dioxane), esters (e.g. ethyl acetate), amides (e.g. N,N-dimethylformamide, N,N-dimethylacetamide), etc. The amount of the optically active thiolocarboxylic acid (IX) may be usually a molar equivalent or slightly excess to the racemic epoxide (VIII).

From the 1:1 diastereomeric mixture, the (R)-isomer (Xb) is separated and collected by a per se conventional separation procedure such as fractional crystallization. For the fractional crystallization, an inert solvent such as an alcohol (e.g. methanol, ethanol, isopropanol) or a hydrocarbon (e.g. n-hexane, benzene, toluene, xylene) may be used.

The most straigtforward procedure for obtaining the desired (R)-isomer (Xb) comprises performing the reaction between the racemic epoxide (VIII) and the optically active thiolocarboxylic acid (IX) in a solvent usable for the fractional crystallization. In such case, the crystals of the (R)-isomer are separated out from the reaction system on the completion of the reaction and can be readily collected by filtration.

The separated (R)-isomer (Xb) is then treated with a base in an inert solvent at a temperature of about −10 to 100°C in an inert atmosphere (e.g. nitrogen, argon) to give the imidazolylthiol of the formula (XI). As the base, there may be used an alkali metal hydroxide (e.g. lithium hydroxide, sodium hydroxide, potassium hydroxide), an alkali metal carbonate (e.g. sodium carbonate, potassium carbonate), an alkali metal alcoholate (e.g. sodium ethylate, sodium methylate), an alkali metal sulfhydrate (e.g. sodium sulfhydrate, potassium sulfhydrate), ammonia, organic amines (e.g. monomethylamine, diethylamine, triethylamine), etc. Examples of the inert solvent are water, alcohols (e.g. methanol, ethanol, isopropanol), ketones (e.g. acetone, methyl ethyl ketone), ethers (diethyl ether, tetrahydrofuran, dioxane), amides (e.g. N,N-dimethylformamide, N,N-dimethylacetamide), etc. Their mixtures are also usable. The amount of the base is usually not less than 1 mole, preferably from 1 to 20 moles, per mole of the (R)-isomer (Xb).

Then, the imidazolylthiol (XI) is reacted with an alkylating agent of the formula (XII), preferably in the presence of a base in an inert solvent to give the objective optically active imidazolylpropanol compounds (I). In the formula (XII) for the alkylating agent, X represents a halogen atom (e.g. chlorine, bromine, iodine), an alkylsulfonyloxy group (e.g. methanesulfonyloxy) or an arylsulfonyloxy group (e.g. benzenesulfonyloxy, toluenesulfonyloxy). Examples of the base are an alkali metal hydroxide (e.g. lithium hydroxide, sodium hydroxide, potassium hydroxide), an alkali metal carbonate (e.g. sodium carbonate, potassium carbonate), an alkali metal (e.g. lithium, sodium, potassium), an alkali metal hydride (e.g. sodium hydride), a tertiary amine (e.g. pyridine, triethylamine), etc. As the inert solvent, there may be used a hydrocarbon (e.g. benzene, toluene, xylene), a chlorinated hydrocarbon (e.g. methylene chloride, chloroform, 1,2-dichloroethane), an alcohol (e.g. methanol, ethanol, isopropanol), a ketone (e.g. acetone, methyl ethyl ketone), an ether (e.g. diethyl ether, tetrahydrofuran, dioxane), an ester (e.g. ethyl acetate) an amide (e.g. N,N-dimethylformamide, N,N-dimethylacetamide), water, etc. The amount of the alkylating agent (XII) may be more than 1 mole, preferably 1 to 10 moles, per mole of the imidazolylthiol (XI). The amount of the base is usually more than 1 mole, preferably 1 to 30 moles, per mole of the imidazolylthiol (XI). The reaction temperature is usually above −20°C, preferably from −20 to 100°C.

The optically active imidazolylpropanol compound (I) can be prepared more conveniently and advantageously by treating the (R)-isomer (Xb) successively with the base and with the alkylating agent (XII) in a single reaction vessel without isolation of the intermediarily produced imidzolylthiol (XI).

The manner for recovery of the optically active imidazolylpropanol compound (I) from the reaction mixture depends upon the property of the reactants as used, but in general, the reaction mixture is subjected to evaporation of the solvent, dilution of the residue with water and extraction of the objective optically active imidazolylpropanol compound (I) with an appropriate water-immiscible solvent.

The thus produced optically active imidazolylpropanol compound (I) may be converted into its acid addition salt by treatment with an acid (e.g. hydrochloric acid, hydrobromic acid, phosphoric acid, nitric acid, acetic acid, maleic acid, succinic acid, fumaric acid, tartaric acid, citric acid, salicylic acid, sorbic acid, lactic acid, oxalic acid).

The optically active imidazolylpropanol compounds of the formula (I) are usually administered to patients orally or parenterally and are ordinarily employed in the form of a pharmaceutical composition which contains them in an effective and non-toxic amount in admixture with conventional pharmaceutical carrier materials suitable for oral or parenteral application and being unreactive with the active compounds. The pharmaceutical composition may be in the dosage form of tablets, capsules, granules, fine granules, powders, syrups, suspensions, emulsions, suppositories, injections, or the like. These pharmaceutical compositions can be prepared by conventional methods by using conventional carrier materials, excipients, binding agents, stabilizers, etc. For injection, the preparation may be prepared by dissolving the active compounds in purified water for injection, which may optionally contain other additives, such as isotonic agents (e.g. glucose, saline), buffering agents, solubilizers, pH adjusting agents or preservatives.

The dosage of the optically active imidazolyl-propanol compounds (I) may vary with the administration routes, the age and weight of the patient, the kinds and severity of the diseases to be treated, or the like. In case of oral administration in adult, it is usually used in an amount of 50 to 1,000 mg, preferably of 100 to 500 mg, per day, which may be administered once a day but may also be divided and administered in two to several times per day. In case of injection in adult, it is usually used in an amount of 10 to 400 mg, preferably 20 to 200 mg, per day, which may be administered once a day but may also be divided and administered in two to several times per day.

Practical and presently preferred embodiments for production of the compounds (I) are illustatively shown in the following Examples.

Example 1

Preparation of (S)-3-[(S)-2-(4-chlorophenyl)isovaleryloxy]-2-(2,4-dichlorophenyl)-1-(imidazol-1-yl)-2-propanol (IVa) and (R)-3-[(S)-2-(4-chlorophenyl)isovaleryloxy]-2-(2,4-dichlorophenyl)-1-(imidazol-1-yl)-2-propanol (IVb):—

To an ice-cooled solution of 2-(2,4-dichlorophenyl;-1-(imidazol-1-yl)-2,3-propanediol (II) (7g, 0.024 mole) and pyridine (2.39 g, 0.03 mole) in tetrahydrofuran (120 ml) was added a solution of (S)-2-(4-chlorophenyl)isovaleryl chloride (III) (6.19 g, 0.027 mole) in tetrahydrofuran (20 ml) in 15 minutes. The resultant mixture was stirred at 0 to 10°C for 1 hour and then at room temperature for 30 minutes. The solvent was removed under reduced pressure, and the residue was extracted with dichloromethane. The extract was washed with a 10% sodium hydroxide solution (20 ml) and water in order, dried over magnesium sulfate and evaporated to give an oily mixture of (S)-3-[(S)-2-(4-chlorophenyl)isovaleryloxy]-2-(2,4-dichlorophenyl)-1-(imidazol-1-yl)-2-propanol (IVa) and (R)-3-[(S)-2-(4-chlorophenyl)isovaleryloxy]-2-(2,4-dichlorophenyl)-1-(imidazol-1-yl)-2-propanol (IVb) (10.1 g). Yield, 93%.

NMR ($CDCl_3$) δ: 0.53 (3H, d, J=7Hz), 0.75 (3H, d, J=7Hz), 1.9—2.3 (1H, m), 2.95 (1H, d, J=10Hz), 4.2—4.6 (2H, m), 4.80 and 4.40 (each 0.5H, d, $J_{gem}$=14Hz), 4.83 and 4.38 (each 0.5H, d, $J_{gem}$=14Hz), 6.7—.5 (10H).

The oily mixture was subjected to high performance low pressure chromatography over silica gel (600 g, Lichroprep® SI 60) eluted with 0.4% (v/v) methanolchloroform. (S)-3-[(S)-2-(4-Chlorophenyl)isovaleryloxy]-2-(2,4-dichlorophenyl)-1-(imidazol-1-yl)-2-propanol (IVa) (3.5 g) was obtained from the first fraction as crystals melting at 145 to 146.5°C. Yield, 34.7%. $[\alpha]_D^{27}$ + 60.5° (c = 1.0, methanol).

Elementary analysis for $C_{23}H_{23}Cl_3N_2O_3$ (%): Calcd.: C, 57.34; H, 4.78; N, 5.82. Found: C, 57.53; H, 4.81; N, 5.75.

(R)-3-[(S)-2-(4-chlorophenyl)isovaleryloxy]-2-(2,4-dichlorophenyl)-1-(imidazol-1-yl)-2-propanol (IVb) (3.1 g) was obtained from the third fraction as crystals melting at 174 to 175°C. Yield, 30.7%. $[\alpha]_D^{27}$ −53.2° (C = 1.0, methanol).

Elementary analysis for $C_{23}H_{23}Cl_3N_2O_3$ (%):
Calcd.: C, 57.34; H, 4.78; N, 5.82. Found: C, 57.29; H, 4.82; N, 5.80.

The second fraction gave a mixture of the compounds (IVa) and (IVb).

## Example 2

Preparation of (R)-3-[(S)-2-(4-chlorophenyl)isovaleryloxy]-2-(2,4-dichlorophenyl)-1-(imidazol-1-yl)-2-propanol (IVb):—

The 1:1 mixture of the compounds (IVa) and (IVb) obtained in Example 1 (46.5 g) was dissolved in acetone-ether (1:2, 130 ml) and stirred at room temperature for 3 hours. The resulting precipitate was collected by filtration, washed with ether and dried to give nearly pure crystals (12.4 g) melting at 174 to 175°C. Recrystallization of the crystals from acetone-ether (2:1) gave (R)-3-[(S)-2-(4-chlorophenyl)isovaleryloxy]-2-(2,4-dichlorophenyl)-1-(imidazol-1-yl)-2-propanol (IVb) (11.46 g) melting at 174 to 175°C as pure crystals.

## Example 3

Preparation of (R)-2-(2,4-dichlorophenyl)-1-(imidazol-1-yl)-2,3-propanediol (V):—

A mixture of (IVb) (6.85 g, 0.015 mole) 95% sodium hydroxide (1.8 g), 95% ethanol (60 ml) and water (10 ml) was stirred at 80°C for 4 hours. The solvent was removed under reduced pressure from the reaction mixture to give an oily residue. The residue was dissolved in 6N hydrochloric acid (70 ml) and washed with chloroform (40 ml). The aqueous layer was neutralized by adding a conc. ammonium hydroxide solution thereto. The precipitate was collected by filtration to give (R)-2-(2,4-dichlorophenyl)-1-(imidazol-1-yl)-2,3-propanediol (V) (3.6 g). Yield, 88%. M.P., 138.5—141°C. $[\alpha]_D^{24}$ − 108.5° (c = 1.0, methanol).

## Example 4

Preparation of (R)-2-(2,4-dichlorophenyl)-2-(imidazol-1-yl)methyloxirane (VI):—

To an ice-cooled solution of (R)-2-(2,4-dichlorophenyl)-1-(imidazol-1-yl)-2,3-propanediol (V) (5.4 g, 0.019 mole) in pyridine (50 ml) was added portionwise methanesulfonyl chloride (2.15 g), and the mixture was stirred at 0 —10°C for 2 hours. A solution of potassium hydroxide (3.7 g, 0.056 mole) in methanol (60 ml) was added thereto at 0—10°C, followed by stirring at 0—10°C for 1 hour. The solvent was removed under reduced pressure below 15°C. The residue was extracted with chloroform, and the extract was dried over magnesium sulfate and evaporated to remove the solvent. The oily residue was purified by silica gel chromatography to give a crystalline mass. Recrystallization from ether-hexane gave (R)-2-(2,4-dichlorophenyl)-2-(imidazol-1-yl)methyloxirane (VI) (4.3 g). Yield, 84.6%. M.P. 107—108.5°C. $[\alpha]_D^{21.5}$ −8.4° (c = 1.0, methanol).

## Example 5

Preparation of (R)-3-(n-butylthio)-2-(2,4-dichlorophenyl)-1-(imidazol-1-yl)-2-propanol (I: n = 3) hydrochloride:—

To a suspension of 60% sodium hydride (0.67 g, 0.017 mole) in dimethoxyethane (40 ml) kept at 0—10°C, a solution of n-butyl mercaptan (1.38 g, 0.015 mole) in dimethoxyethane (20 ml) was added, and the mixture was stirred for 10 minutes. A solution of (R)-2-(2,4-dichlorophenyl)-2-(imidazol-1-yl)methyloxirane (VI) (3.7 g, 0.014 mole) in dimethoxyethane (20 ml) was added thereto, followed by stirring at room temperature for 2 hours. The solvent was removed, and the residue was extracted with chloroform. The chloroform extract was washed with water, dried and evaporated to remove the solvent. The oily residue was dissolved in ether, and a solution of hydrogen chloride gas in ether was added thereto. The precipitated crystals were collected by filtration and recrystallized from a mixture of methanol and ether to give (R)-3-(n-butylthio)-2-(2,4-dichlorophenyl)-1-(imidazol-1-yl)-2-propanol (I: n = 3) hydrochloride (4.65 g). Yield, 85.5% M.P., 164—165°C. $[\alpha]_D^{20}$ −89.3° (C = 1.0, methanol).

## Example 6

Preparation of (R)-3-(n-butylthio)-2-(2,4-dichlorophenyl)-1-(imidazol-1-yl)-2-propanol hydrochloride:—

To a solution of (R)-2-(2,4-dichlorophenyl)-1-(imidazol-1-yl)-2,3-propanediol (V) (2.87 g, 0.01 mole) and triethylamine (1.21 g, 0.012 mole) in dichloromethane (30 ml) kept at 0—5°C was added methanesulfonyl chloride (1.37 g, 0.012 mole), and the mixture was stirred at 0—5°C for 1 hour. The reaction mixture was poured into water and extracted with dichloromethane. The extract was washed with water, dried over magnesium sulfate and evaporated to give the mesylate as an oil (4.2 g). To a suspension of 60% sodium hydride (0.6 g, 0.015 mole) in tetrahydrofuran (20 ml) kept at 0—5°C was added n-butyl mercaptan (1.35 g, 0.015 mole), and the mixture was stirred at 0—5°C for 30 minutes. A solution of the oily mesylate (4.2 g) in tetrahydrofuran (40 ml) was added thereto, followed by stirring at room temperature for 1 hour. The solvent was removed below 25°C, and the residue was poured into water and extracted twice with 1,2-dichloroethane (30 ml). The 1,2-dichloroethane extract was washed with water, dried and evaporated to give an oil. The oil was dissolved in ether and treated with hydrogen chloride gas. The precipitated crystals (2.8 g) were collected by filtration and recrystallized from methanol-ether to give (R)-3-(n-butylthio)-2-(2,4-dichlorophenyl)-1-(imidazol-1-yl)-2-propanol (I: n = 3) hydrochloride (2.3 g). Yield, 58.1%. M.P., 164—165°C. $[\alpha]_D^{20}$ −89.3° (C = 1.0, methanol).

Example 7

Preparation of (R)-2-(2,4-dichlorophenyl)-1-(imidazol-1-yl)-3-(n-pentylthio)-2-propanol (I: n = 4) oxalate:—

(R)-2-(2,4-Dichlorophenyl)-1-(imidazol-1-yl)-3-(n-pentylthio)-2-propanol (I: n = 4) was obtained by using n-pentyl mercaptan instead of n-butyl mercaptan in Example 5. The free base was treated with oxalic acid to give the oxalate. M.P., 130—133.5°C. $[\alpha]_D^{24}$ −73.3°C (c = 1.0, methanol).

Reference Example 1

Preparation of (S)-2-(2,4-dichlorophenyl)-1-(imidazol-1-yl)-2,3-propanediol:—

A mixture of (S)-3-[(S)-2-(4-dichlorophenyl)isovaleryloxy)-2-(2,4-dichlorophenyl)-1-(imidazol-1-yl)-2-propanol (IVa) (2.89 g, 0.006 mole) obtained in Example 1, a 20% aqueous potassium hydroxide solution (6 ml) and methanol (40 ml) was stirred at room temperature for 3 hours. The solvent was removed to give an oily residue. The residue was dissolved in 6N hydrochloric acid (30 ml) and washed with chloroform (40 ml). The aqueous layer was neutralized by adding a conc. ammonium hydroxide solution thereto. The resulting precipitate was collected by filtration to give (S)-2-(2,4-dichlorophenyl)-1-(imidazol-1-yl)-2,3-propanediol (1.51 g). Yield, 88%. M.P. 143°C. $[\alpha]_D^{19}$ + 113.1° (c = 1.0 methanol).

Reference Example 2

Preparation of (S)-2-(2,4-dichlorophenyl)-2-(imidazol-1-yl)methyloxirane:—

To an ice-cooled solution of (S)-2-(2,4-dichlorophenyl)-1-(imidazol-1-yl)-2,3-propanediol (2.5 g, 0.009 mole) in pyridine (30 ml) was added portionwise methanesulfonyl chloride (1.05 g, 0.009 mole), and the mixture was stirred at 0—5°C for 6 hours. A solution of potassium hydroxide (1.46 g) in methanol (30 ml) was added thereto at 0—5°C, followed by stirring at 0—5°C for 1 hour and then at 20°C for 1 hour. The solvent was removed below 20°C to give an oily residue. The oily residue was poured into water and extracted with chloroform-ether (5 : 1). The extract was dried over magnesium sulfate and evaporated to remove the solvent. The residue was purified by silica gel chromatography to give an oil. Treatment of the oil with ether-hexane gave (S)-2-(2,4-dichlorophenyl)-2-(imidazol-1-yl)methyloxirane (1.94 g). Yield, 82.9%. M.P., 107—108.5°C $[\alpha]_D^{24}$ + 8.8° (c = 1.0, methanol).

Reference Example 3

Preparation of (S)-3-(n-butylthio)-2-(2,4-dichlorophenyl)-1-(imidazol-1-yl)-2-propanol hydrochloride:—

To a suspension of 60% sodium hydride (1.07 g, 0.027 mole) in dimethoxyethane (40 ml) kept at 10—20°C, a solution of n-butyl mercaptan (2.20 g, 0.024 mole) in dimethoxyethane (20 ml) was added, and the mixture was stirred for 1 hour. A solution of (S)-2-(2,4-dichlorophenyl)-2-(imidazol-1-yl)methyloxirane (3.03 g, 0.014 mole) in dimethoxyethane (20 ml) was added thereto, followed by stirring at 0—15°C for 1 hour and then at room temperature for 1 hour. The solvent was removed, and the residue was poured into water and extracted with chloroform. The chloroform extract was washed with water, dried and evaporated to give an oil. The oil was dissolved in ether and a solution of hydrogen chloride gas in ether was added thereto. The precipitated crystals were collected by filtration, treated with charcoal and recrystallized from methanol-ether to give (S)-3-(n-butylthio)-2-(2,4-dichlorophenyl)-1-(imidazol-1-yl)-2-propanol hydrochloride (4.0 g). Yield, 89.9%. M.P. 165—166°C $[\alpha]_D^{24}$ + 88.9° (c = 1.0, methanol).

Reference Example 4

Preparation of (S)-2-(2,4-dichlorophenyl)-1-(imidazol-1-yl)-3-(n-pentylthio)-2-propanol oxalate:—

(S)-2-(2,4-Dichlorophenyl)-1-(imidazol-1-yl)-3-(n-pentylthio)-2-propanol was obtained by using n-pentyl mercaptan instead of n-butyl mercaptan in Reference Example 3. The free base was treated with oxalic acid to give the oxalate. M.P., 132—134.5°C $[\alpha]_D^{24}$ + 73.9° (c = 1.0, methanol).

Example 8

Preparation of (R)-3-[(S)-2-(4-chlorophenyl)isovalerylthio]-2-(2,4-dichlorophenyl)-1-(imidazol-1-yl)-2-propanol (Xb):—

To a solution of dl-2-(2,4-dichlorophenyl)-2-(imidazol-1-yl)methyloxirane (VIII) (269 g) in toluene (1100 ml) was added (S)-2-(4-chlorophenyl)thioloisovaleric acid (IX) (229 g) obtained in Reference Example 5 at room temperature. The mixture was stirred without external cooling, while the temperature raised spontaneously to 55°C during the first 10 minutes. The mixture was kept at the same temperature as above for another 1 hour and then at 20°C for 3 hours. The precipitate was collected by filtration, washed successively with toluene and isopropanol and dried in vacuo to give (R)-3-[(S)-2-(4-chlorophenyl)-isovalerylthio]-2-(2,4-dichlorophenyl)-1-(imidazol-1-yl)-2-propanol (Xb) (204 g). Yield, 41%. M.P., 182—183°C. $[\alpha]_D^{23}$ −1.0° (c = 1, methanol).

Example 9

Preparation of (R)-2-(2,4-dichlorophenyl)-1-(imidazol-1-yl)-3-mercapto-2-propanol (XI):—

To a suspension of the compound (Xb) (49.5 g) obtained in Example 8 in toluene (300 ml) was added a 10% methanolic potassium hydroxide solution (160 g) at a temperature of −5°C to 0°C in nitrogen atmosphere. The mixture was warmed to 25°C over a period of about 30 minutes and stirred at the same temperature for 2 hours. The reaction mixture was concentrated under reduced pressure. The residue was dissolved in dichloromethane (300 ml), washed with water, dried and evaporated to give an oil. The oil was purified by silica gel column chromatography using chloroform as an eluent and recrystallized from a mixture of dichloromethane and n-hexane to give (R)-2-(2,4-dichlorophenyl)-1-(imidazol-1-yl)-3-mercapto-2-propanol (XI) (9.4 g). Yield, 31%. M.P., 155—157°C $[\alpha]_D^{24}$ −6.6° (c = 1, methanol).

Example 10

Preparation of (R)-2-(2,4-dichlorophenyl)-1-(imidazol-1-yl)-3-mercapto-2-propanol (XI):—

In the same manner as in Example 9 but using 10% water-containing methanol (400 ml) and sodium sulfhydrate (10 g) in place of methanol and potassium hydroxide, respectively, there was obtained (R)-2-(2,4-dichlorophenyl)-1-(imidazol-1-yl)-3-mercapto-2-propanol (XI) (11.3 g). Yield, 37% M.P., 156—157°C $[\alpha]_D^{24}$ −7.0° (c = 1, methanol).

Example 11

Preparation of (R)-3-(n-butylthio)-2-(2,4-dichlorophenyl)-1-(imidazol-1-yl)-2-propanol (I: n = 3) hydrochloride:—

To a solution of sodium hydroxide (2 g) in methanol (100 ml) were added n-butylbromide (2 g) and (R)-2-(2,4-dichlorophenyl)-1-(imidazol-1-yl)-3-mercapto-2-propanol (XI) obtained in Example 9 at a temperature of 20—25°C in nitrogen atmosphere. The mixture was stirred at 25—30°C for 5 hours and then concentrated under reduced pressure. The residue was treated with water (50 ml) and extracted with dichloromethane (50 ml). The extract was washed with water, dried over anhydrous magnesium sulfate and concentrated to give an oil. The oil was dissolved in diethyl ether (20 ml), and hydrogen chloride gas was introduced thereto. Precipitated crystals were collected by filtration and dried to give (R)-3-(n-butylthio)-2-(2,4-dichlorophenyl)-1-(imidazol-1-yl)-2-propanol (I: n = 3) hydrochloride (3.4 g). Yield, 86.8%. m.p., 168—169°C. $[\alpha]_D^{20}$ −89.7° (c = 1, methanol). The optical purity of the product was not less than 99% determined by high performance liquid chromatography.

Example 12

Preparation of (R)-3-(n-butylthio)-2-(2,4-dichlorophenyl)-1-(imidazol-1-yl)-2-propanol (I: n = 3) hydrochloride:—

To a solution of (R)-2-(2,4-dichlorophenyl)-1-(imidazol-1-yl)-3-mercapto-2-propanol (IX) (3 g) in dry N,N-dimethylformamide (20 ml) were added a 50% dispersion of sodium hydride in paraffin (1 g) and n-butyl p-toluene-sulfonate (2.3 g) at 0—5°C in nitrogen atmosphere. The mixture was stirred at 0—5°C for 1 hour and at 20—25°C for 8 hours. The reaction mixture was diluted with water (200 ml) and extracted with dichloromethane. Treatment of the extract in the same manner as in Example 11 gave (R)-3-(n-butylthio)-2-(2,4-dichlorophenyl)-1-(imidazol-1-yl)-2-propanol (I: n = 3) hydrochloride (2.5 g). Yield, 63.8%. M.P., 168—169°C. $[\alpha]_D^{20}$ −88.8° (c = 1, methanol).

Example 13

Preparation of (R)-3-(n-butylthio)-2-(2,4-dichlorophenyl)-1-(imidazol-1-yl)-2-propanol (I: n = 3) hydrochloride:—

To a suspension of (R)-3-[(S)-2-(4-chlorophenyl)isovalerylthio]-2-(2,4-dichlorophenyl)-1-(imidazol-1-yl)-2-propanol (Xb) (49.5 g) obtained in Example 8 in methanol (500 ml) were added a 10% methanolic potassium hydroxide solution (240 g) and n-butylbromide (21.6 g) at −5 to 0°C in nitrogen atmosphere. The slurry was stirred at the same temperature for 2 hours and then at 30°C for 3 hours. Methanol was removed by distillation under atmospheric pressure, and the residue was treated with water (200 ml) and extracted with 1,2-dichloroethane (200 ml). The extract was washed successively with water and 12% hydrochloric acid (200 ml) and concentrated in vacuo. To the residue were added toluene (100 ml) and methyl ethyl ketone (35 ml), and the slurry was stirred at 15°C for 3 hours. Filtration and drying in vacuo gave a crude product (25.7 g), which was recrystallized from methyl ethyl ketone to give (R)-3-(n-butylthio)-2-(2,4-dichlorophenyl)-1-(imidazol-1-yl)-2-propanol (I: n = 3) hydrochloride (21.8 g) as colorless needles. Yield, 55%. M.P., 168.5—170°C. $[\alpha]_D^{20}$ −89.8° (C = 1, methanol).

Reference Example 5

Preparation of (S)-2-(4-chlorophenyl)thioloisovaleric acid (IX):—

To a solution of potassium hydroxide (296 g) in methanol (1500 ml) was introduced hydrogen sulfide gas (180 g) from a gas-inlet tube at a temperature below 30°C, and the mixture was cooled at −5°C. A solution of (S)-2-(4-chlorophenyl)oisovaleryl chloride (510 g) in toluene (1000 ml) was dropwise added thereto at a temperature of −5 to 10°C. The mixture was stirred at the same temperature for 30 minutes, followed by addition of water (2000 ml). A 35% hydrochloric acid (455 g) was dropwise added thereto. The

toluene layer was washed with water (500 ml) and concentrated in vacuo to give (S)-2-(4-chlorophenyl)thioloisovaleric acid (IX) (504 g), which was used for the reaction in Example 8 without purification.

The optically active imidazolylpropanols (I) exhibit a remarkable antimicrobial activity against various microorganisms, particularly fungi. Also, some of them show an antifungal activity against phytopathogenic fungi. In the in vitro and in vivo tests, the antifungal activity of the representative optically active imidazolylpropanol (I: n = 3) (i.e. (R)-isomer) was compared with those of the corresponding racemate (Compound A) and the corresponding (S)-isomer (Compound B).

Experiment 1

In vitro test of the compound (I):—

Method

Candida albicans, strain KB—8 (Clinical isolate), was maintained on Sabouraud's agar slants at room temperature. Yeast cells were grown at 37°C on Sabouraud's agar and a loopful from a 24 hours' slant was inoculated in 4.5 ml of Sabouraud's broth and cultured at 37°C for 24 hours. A 0.5 ml aliquot of this culture was inoculated in 100 ml of a casein hydrolysate yeast-extract glucose medium (CYG medium). Cells were grown at 37°C aerobically in a reciprocating shaker for 48 hours. From this culture, a 0.2 ml aliquot was again inoculated in 100 ml CYG medium and grown for another 24 hours at 37°C while shaking.

The pseudomycelium growth-promoting medium consisted of Eagle's minimal essential medium supplemented with non-essential amino acids and sodium bicarbonate (2 g/liter). Yeast cells were seeded at densities of $1.0 \times 10^5$/ml in sterile culture tubes ($\phi 10 \times 100$ mm) containing 3 ml of medium. Cells were grown in a humidified atmosphere of 5% $CO_2$ in air at 37°C.

The effects of the test compound were evaluated under the following conditions: the cultures were exposed to the test compound in doses ranging from $10^{-10}$ M to $10^{-4}$ M; the solvent for the test compound was added as an aqueous solution. Pseudomycelium development was observed by light microscopy at 72 hours after inoculation.

The effective doses to inhibit the formation of pseudomycelium are shown in Table 1.

TABLE 1
Antifungal activity in vitro

| | Imidazolylpropanol compound | Minimum effective dose |
|---|---|---|
| I (n=3) | 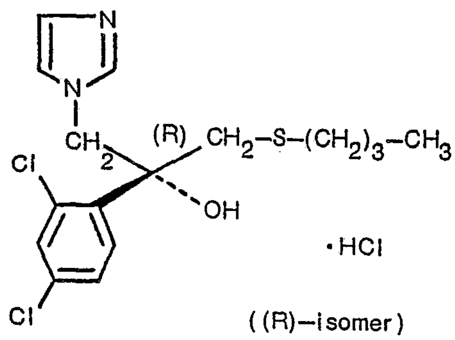 • HCl ((R)—isomer) | $10^{-9}$ M |
| A | 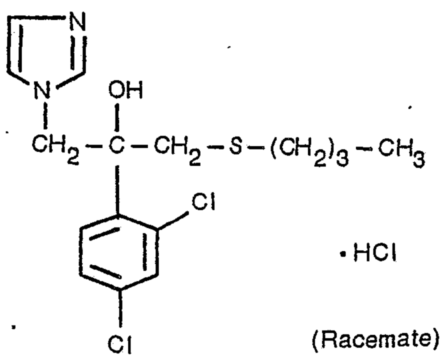 • HCl (Racemate) | $10^{-7}$ M |
| B | 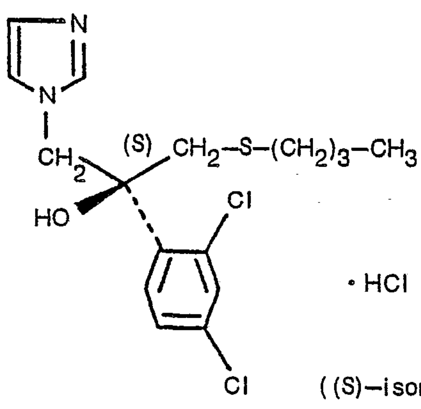 • HCl ((S)—isomer) | $10^{-6}$ M |

Experiment 2

In vivo test of the compound (I):—

(1) Effects on the number of viable cells in the kidney of Candida-infected mice.

Method

Candida albicans KB—8 was cultured on a Sabouraud's agar plate admixed with blood in a concentration of 5% (v/v) at 30°C for 4 days, suspended in physiologically saline solution and diluted to approximately 10,000,000 cells/ml. DDY strain male mice were insulated via the tail vein with 0.2 ml of this suspension.

The animals were orally medicated with the test compound in the form of 0,5% (w/v) methylcellulose suspension at a total dose of 20 mg per kg of the body weight immediately and 5 hours after the infection.

After 24 hours from the infection, the animals were sacrificed, and the kidneys were taken out. After the kidneys were homogenized in 4 fold sterilized physiologically saline solution, a certain amount of the suspension was scattered on a Sabouraud's agar plate and cultured at 37°C for 48 hours. Then, the number of colonies was counted, the number of cells per one gram of the kidney was calculated, and the difference between the calculated numbers in the medicated group and in the non-medicated group was expressed by a logarithmic value. The results are shown in Table 2.

TABLE 2
Antifungal activity in vivo (Decrease of cells in the kidney of Candida-infected mice)

| | Imidazolylpropanol compound | Dose (per os, mg/kg) | Medicated group, number of cells in kidney (cell/g) | Non-medicated group, number of cells in kidney (cell/g) | Medicated/ Non-medicated (logarithmic value) |
|---|---|---|---|---|---|
| I (n=3) | 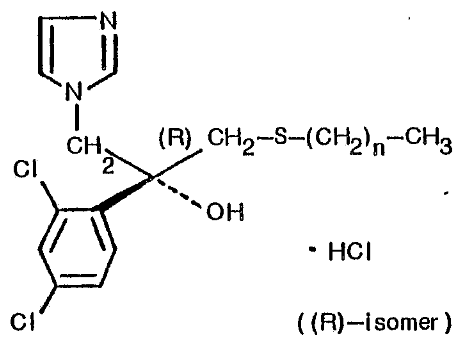 · HCl ((R)–Isomer) | 20 | $1.41\times10^{3}$ | $1.45\times10^{5}$ | −2.01 |
| A (n=3) | 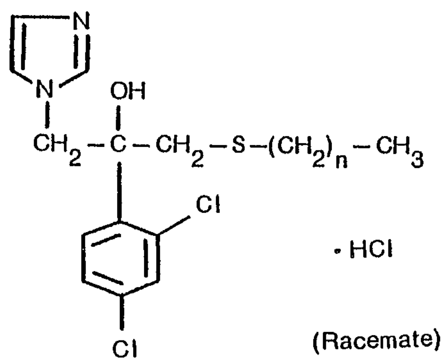 · HCl (Racemate) | 20 | $1.86\times10^{3}$ | $1.45\times10^{5}$ | −1.89 |

TABLE 2 (continued)
Antifungal activity in vivo (Decrease of cells in the kidney of Candida-infected mice)

| | Imidazolylpropanol compound | Dose (per os, mg/kg) | Medicated group, number of cells in kidney (cell/g) | Non-medicated group, number of cells in kidney (cell/g) | Medicated/ Non-medicated (logarithmic value) |
|---|---|---|---|---|---|
| B (n=3) | 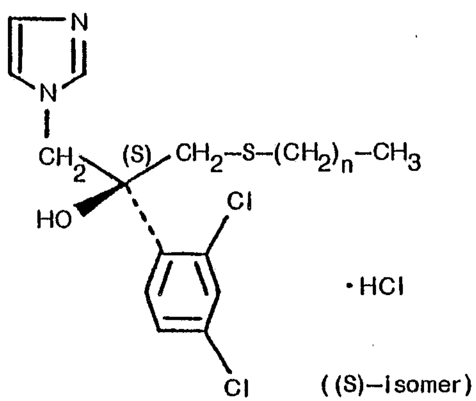 •HCl ((S)–isomer) | 20 | $1.82\times10^4$ | $1.45\times10^5$ | −0.90 |

(2) Effects of decreasing mortality in Candida-infected mice

Method

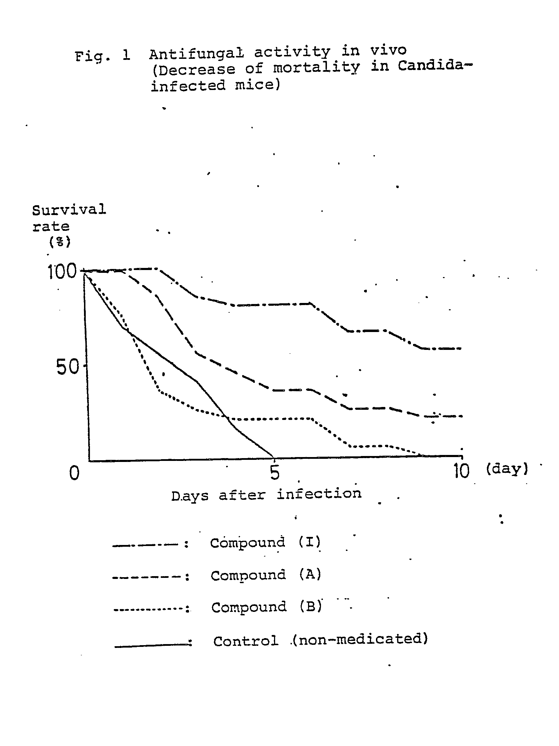

The numbers of survival mice wee recorded for ten days after infection and medication as in Experiment 2 (1). The rate (%) of survival mice per total mice in each group was shown in Figure 1 of the accompanying drawing.

As understood from the above results, the optically active imidazolylpropanol compound (I) of the invention shows an excellent antifungal activity.

Advantageously, the optically active imidazolylpropanol compounds (I) are quite low in toxicity, and their $LD_{50}$ values are more than 500 mg/kg when determined by oral route to mice. Thus, they are useful as antifungal agents.

The optically active imidazolylpropanol compounds (I) can be administered parenterally, orally or locally to warm-blooded animals and human beings in the form of conventional pharmaceutical preparations. For instance, they can be administered in the form of conventional solid pharmaceutical preparations such as tablets, capsules, powders or granules, or in the form of conventional liquid pharmaceutical preparations such as suspensions, emulsions or solutions. The daily dosage may vary depending upon the administration route and is usually between 10 mg and 5 g for human beings.

**Claims for the Contracting States: BE CH DE FR GB IT LI NL SE**

1. An optically active imidazolylpropanol compound of the formula:

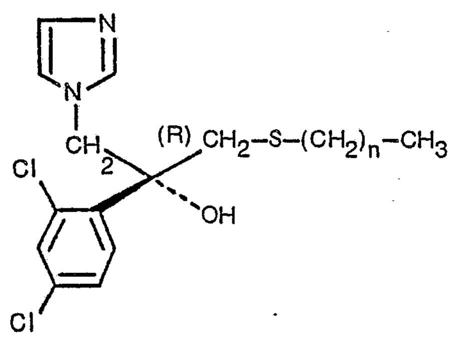

(I)

wherein n is an integer of 3 or 4, and its acid addition salts.

2. The compound according to claim 1, which is (R)-3-(n-butylthio)-2-(2,4-dichlorophenyl)-1-(imidazol-1-yl)-2-propanol.

3. The compound according to claim 1, which is (R)-2-(2,4-dichlorophenyl)-1-(imidazol-1-yl)-3-(n-pentylthio)-2-propanol.

4. A process for preparing optically active imidazolylpropanol compounds according to claim 1 which comprises reacting an epoxide of the formula:

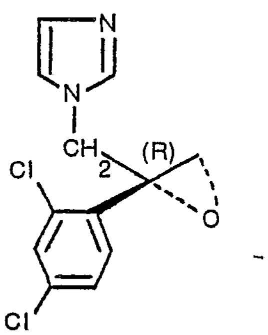

(VI)

with a thiol compound of the formula:

$$HS{-}(CH_2)_n{-}CH_3 \qquad (VII)$$

wherein n is as defined above in a metallized form.

5. A process for preparing optically active imidazolylpropanol compounds according to claim 1 which comprises:

(1) reacting a racemic diol of the formula:

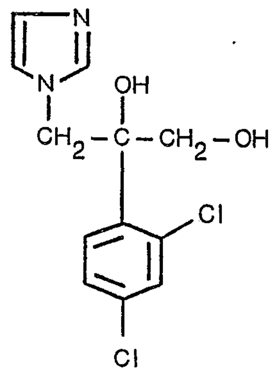

(II)

with an optically active carboxylic acid chloride of the formula: ACl wherein A is an optically active acyl group to form a mixture of two diastereomeric isomers of the formulas:

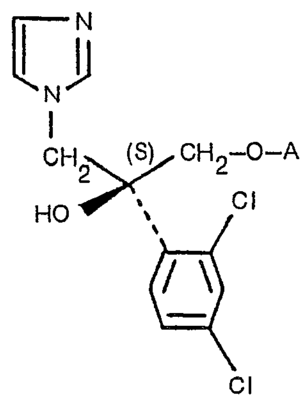

(IVa)

and

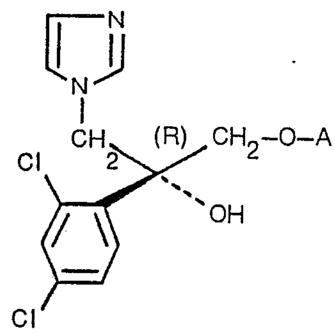

(IVb)

wherein A is as defined above;

(2) separating the (R)-isomer from said mixture;

(3) hydrolyzing the (R)-isomer to give a diol of the formula:

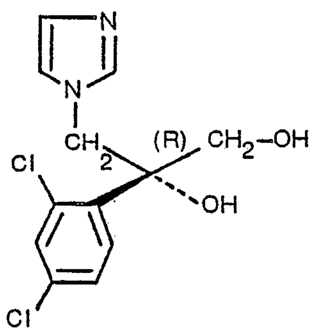

(V)

(4) reacting the diol with methanesulfonyl chloride, followed by treating with a base to give an epoxide of the formula:

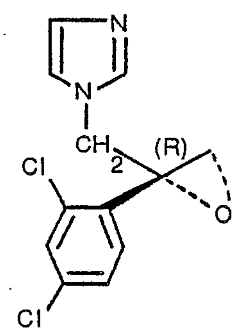

(VI)

and

(5) reacting the epoxide (VI) with a thiol compound of the formula:

$$HS-(CH_2)_n-CH_3 \qquad (VII)$$

wherein n is as defined above in a metallized form.

6. The process according to claim 5, wherein the optically active carboxylic acid chloride is (S)-2-(4-chlorophenyl)isovaleryl chloride, (+)-α-methoxy-α-trifluoromethylphenylacetyl chloride or L-menthoxyacetyl chloride.

7. (R)-2-(2,4-Dichlorophenyl)-1-(imidazol-1-yl-2,3-propanediol of the formula:

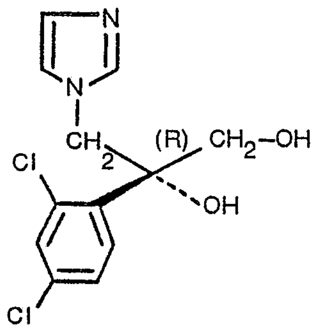

(V)

8. (R)-2-(2,4-Dichlorophenyl)-1-(imidazol-1-yl)-methyloxirane of the formula:

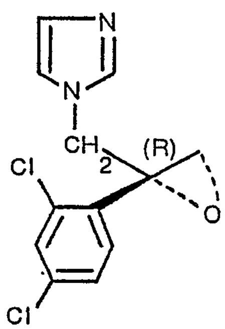

(VI)

9. A process for preparing optically active imidazolylpropanol compounds according to Claim 1 which comprises reacting an optically active imidazolylthiol compound of the formula:

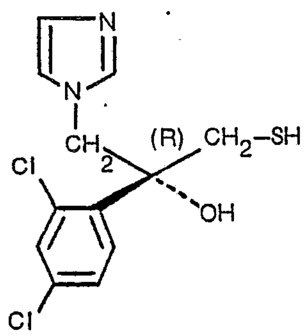

(XI)

with an alkylating agent of the formula:

$$CH_3-(CH_2)_n-X \qquad (XII)$$

wherein n is as defined above and X is a halogen atom, an alkylsulfonyloxy group or an arylsulfonyloxy group.

10. The process according to claim 9, wherein the reaction is carried out in the presence of a base.

11. A process for preparing optically active imidazolylpropanol compounds according to claim 1 which comprises

(1) reacting a racemic epoxide of the formula:

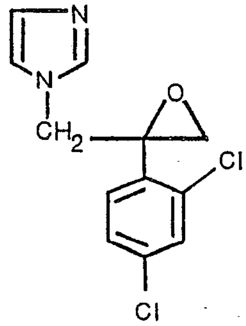

(VIII)

with an optically active thiolocarboxylic acid of the formula: A—SH wherein A is an optically active acyl group to give a mixture of two diastereomeric isomers of the formulas:

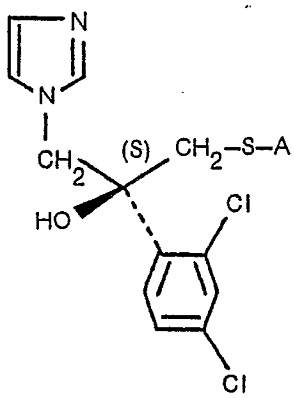

and

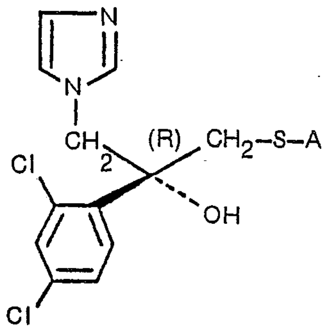

(Xb)

(Xa)

wherein A is as defined above;

(2) separating the (R)-isomer from said mixture;

(3) reacting the (R)-isomer with a base to give an imidazolylthiol of the formula:

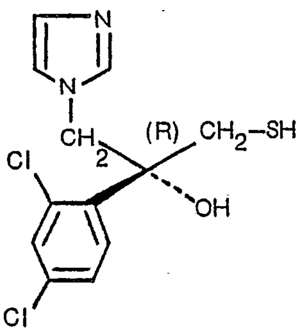

(XI)

and

(4) reacting the imidazolylthiol with an alkylating agent of the formula:

$$CH_3—(CH_2)_n—X \qquad (XII)$$

wherein n is as defined above and X is a halogen atom, an alkylsulfonyloxy group or an arylsulfonyloxy group.

12. The process according to claim 11, wherein the optically active thiolocarboxylic acid is a compond of the formula:

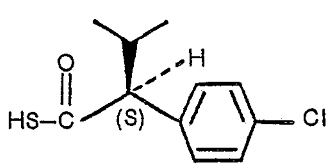

(IX)

13. An antifungal composition which comprises an antifungally effective amount of at least one of the optically active imidazolylpropanol compounds and their acid-addition salts as claimed in claim 1 as an active ingredient and a pharmaceutically acceptable carrier or diluent.

**Claims for the Contracting State: AT**

1. A process for preparing optically active imidazolylpropanol compounds of the formula:

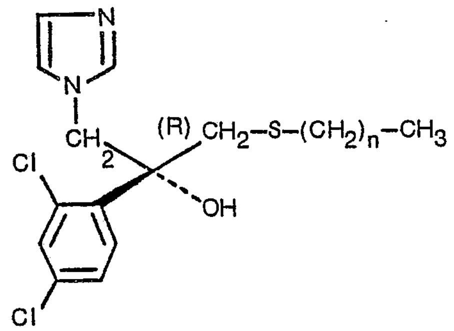

(I)

wherein n is an integer of 3 or 4, and their acid addition salts, which comprises reacting an epoxide of the formula:

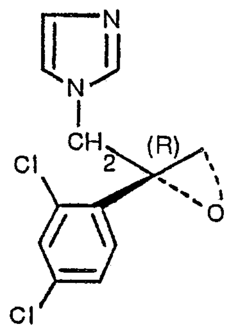

(VI)

with a thiol compound of the formula:

$$HS—(CH_2)_n—CH_3 \qquad (VII)$$

wherein n is as defined above in a metallized form.

2. A process for preparing optically active imidazolylpropanol compounds according to claim 1 which comprises:

(1) reacting a racemic diol of the formula:

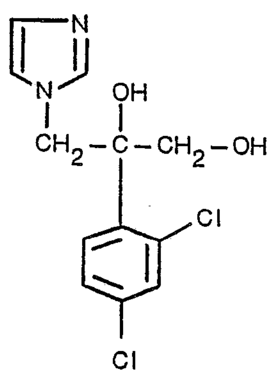

(II)

with an optically active carboxylic acid chloride of the formula: ACl wherein A is an optically active acyl group to form a mixture of two diastereomeric isomers of the formulas:

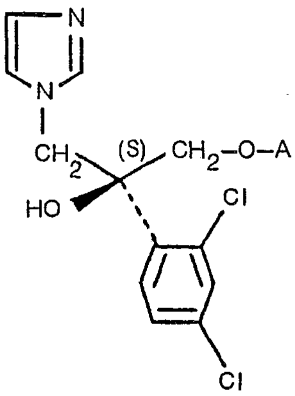

and

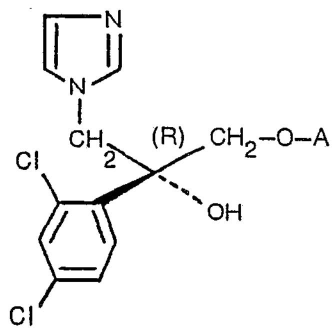

(IVb)

(IVa)

wherein A is as defined above;

(2) separating the (R)-isomer from said mixture;

(3) hydrolyzing the (R)-isomer to give a diol of the formula:

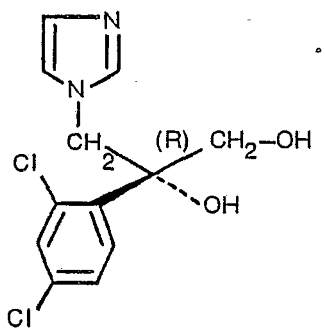

(V)

(4) reacting the diol with methanesulfonyl chloride, followed by treating with a base to give an epoxide of the formula:

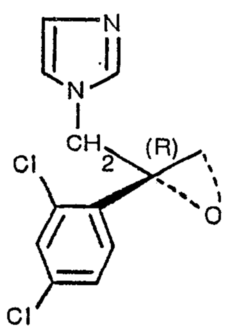

(VI)

and

(5) reacting the epoxide (VI) with a thiol compound of the formula:

$$HS—(CH_2)_n—CH_3 \qquad (VII)$$

wherein n is as defined above in a metallized form.

3. The process according to claim 2, wherein the optically active carboxylic acid chloride is (S)-2-(4-chlorophenyl)isovaleryl chloride, (+)-α-methoxy-α-trifluoromethylphenylacetyl chloride or L-menthoxyacetyl chloride.

4. A process for preparing optically active imidazolylpropanol compounds according to claim 1, which comprises reacting an optically active imidazolylthiol compound of the formula:

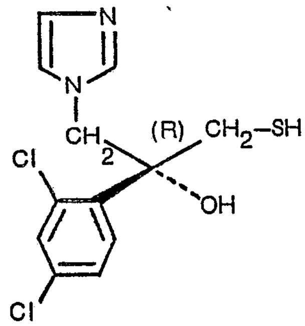

(XI)

with an alkylating agent of the formula:

$$CH_3-(CH_2)_n-X \quad (XII)$$

wherein n is as defined above and X is a halogen atom, an alkylsulfonyloxy group or an arylsulfonyloxy group.

5. The process according to claim 4, wherein the reaction is carried out in the presence of a base.

6. A process for preparing optically active imidazolylpropanol compounds according to claim 1, which comprises:

(1) reacting a racemic epoxide of the formula:

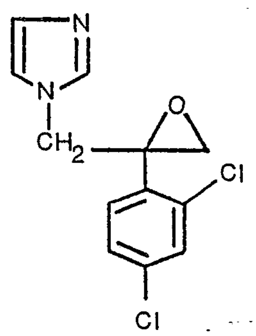

(VIII)

with an optically active thiolocarboxylic acid of the formula: A—SH wherein A is an optically active acyl group to give a mixture of two diastereomeric isomers of the formulas:

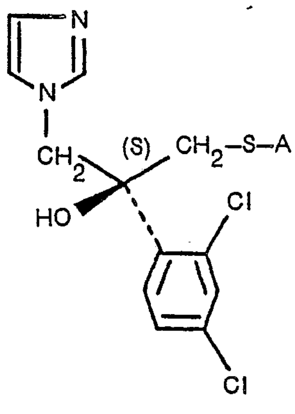

(Xa)

and

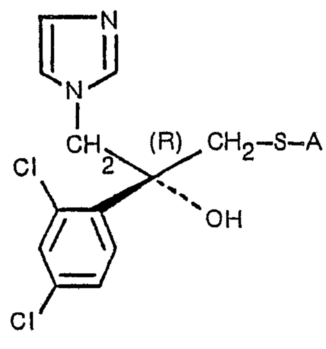

(Xb)

wherein A is as defined above;

(2) separating the (R)-isomer from said mixture;

(3) reacting the (R)-isomer with a base to give an imidazolylthiol of the formula:

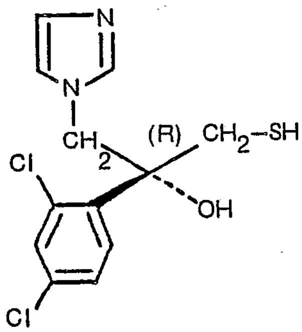

(XI)

and

(4) reacting the imidazolylthiol with an alkylating agent of the formula:

$$CH_3-(CH_2)_n-X \quad (XII)$$

wherein n is as defined above and X is a halogen atom, an alkylsulfonyloxy group or an arylsulfonyloxy group.

7. The process according to claim 6, wherein the optically active thiolocarboxylic acid is a compond of the formula:

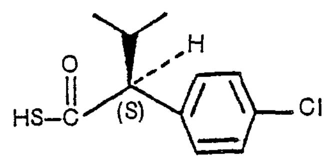

(IX)

8. A process according to claims 1 to 7 for preparing (R)-3-(n-butylthio)-2-(2,4-dichlorophenyl)-1-(imidazol-1-yl)-2-propanol.

9. A process according to claims 1 to 7 for preparing (R)-2-(2,4-dichlorophenyl)-1-(imidazol-1-yl)-3-(n-pentylthio)-2-propanol.

10. A process for preparing an antifungal composition which comprises the formulation of an antifungally effective amount of at least one of the optically active imidazolylpropanol compounds and their acid-addition salts as claimed in claim 1 as an active ingredient and a pharmaceutically acceptable carrier or diluent.

**Patentansprüche für die Vertragsstaaten: BE CH DE FR GB IT LI NL SE**

1. Optisch aktive Imidazolylpropanolverbindung der Formel

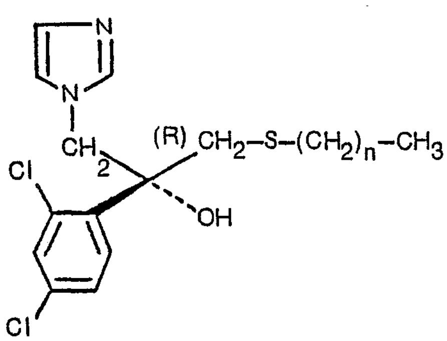

(I).

worin n eine ganze Zahl von 3 oder 4 ist, und ihre Säureadditionssalze.

2. Verbindung nach Anspruch 1, welche (R)-3-(n-Butylthio)-2-(2,4-dichlorphenyl)-1-(imidazol-1-yl)-2-propanol ist.

3. Verbindung nach Anspruch 1, welche (R)-2-(2,4-Dichlorphenyl)-1-(imidazol-1-yl)-3-(n-pentylthio)-2-propanol ist.

4. Verfahren zur Herstellung optisch aktiver Imidazolylpropanolverbindungen nach Anspruch 1, welches die Umsetzung eines Epoxids der Formel

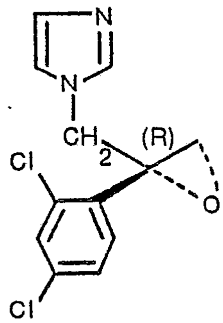

(VI)

mit einer Thiolverbindung der formel

$$HS{-}(CH_2)_n{-}CH_3 \qquad (VII)$$

worin n wie oben definiert ist, welche in einer metallisierten Form vorliegt, umfaßt.

5. Verfahren zur Herstellung optisch aktiver Imidazolylpropanolverbindungen nach Anspruch 1, welches

(1) die Umsetzung eines racemischen Diols der Formel

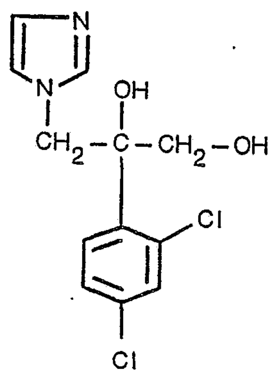

(II)

mit einem optisch aktiven Carbonsäurechlorid der Formel ACl, worin A eine optisch aktive Acylgruppe darstellt, zur Bildung einer Mischung von zwei diastereomeren Isomeren der Formeln

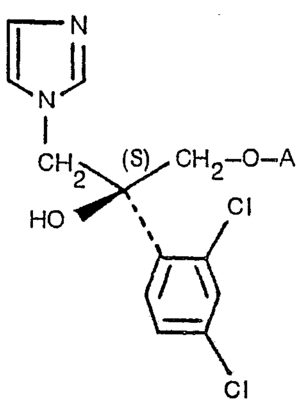

und

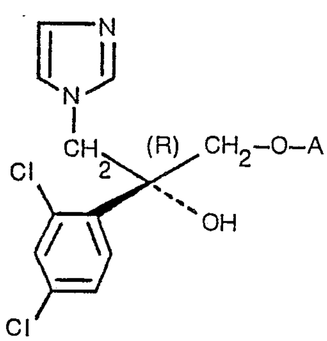

(IVb)

(IVa)

worin A die obige Bedeutung hat;

(2) die Abtrennung des (R)-Isomeren aus dieser Mischung;

(3) die Hydrolyse des (R)-Isomeren zur Erzielung eines Diols der Formel

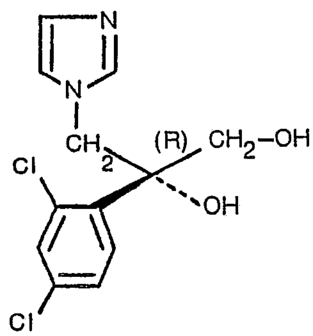

(V)

(4) die Umsetzung des Diols mit Methansulfonylchlorid, gefolgt von Behandlung mit einer Base zur Bildung eines Epoxids der Formel

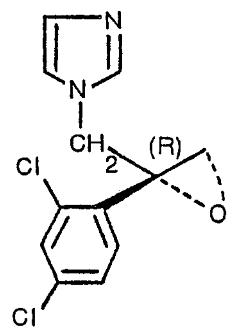

(VI)

und

(5) die Umsetzung des Epoxids (VI) mit einer Thiolververbindung der Formel

$$HS-(CH_2)_n-CH_3 \quad \text{(VII)}$$

worin n wie oben definiert ist, welche in metallisierter Form vorliegt; umfaßt.

6. Verfahren nach Anspruch 5, worin das optisch aktive Carbonsäurechlorid (S)-2-(4-Chlorphenyl)-isovalerylchlorid,(+)-α-Methoxy-α-trifluor-methylenphenylacetylchlorid oder L-Menthoxyacetylchlorid ist.

7. (R)-2-(2,4-Dichlorphenyl)-1-(imidazol-1-yl)-2,3-propandiol der Formel

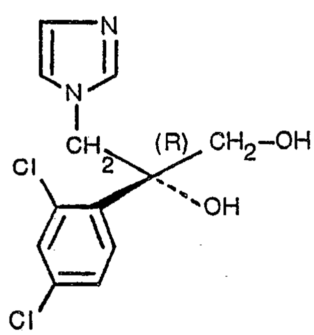

(V)

8. (R)-2-(2,4-Dichlorophenyl)-1-(imidazol-1-yl)-methyloxiran der Formel

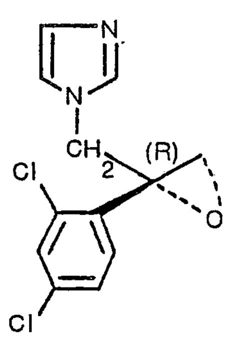

(VI)

9. Verfahren zur Herstellung optisch aktiver Imidazolylpropanolverbindungen nach Anspruch 1, welches die Umsetzung einer optisch aktiven Imidazolylthiol verbindung der Formel

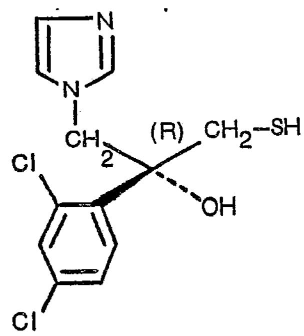

(XI)

mit einem Alkylierungsmittel der Formel

$$CH_3—(CH_2)_n—X \quad (XII),$$

worin n wie oben definiert ist und X für ein Halogenatom, eine Alkylsulfonyloxygruppe oder eine Arylsulfonyloxygruppe steht, umfaßt.

10. Verfahren nach Anspruch 9, worin die Umsetzung in Gegenwart einer Base durchgeführt wird.

11. Verfahren zur Herstellung optisch aktiver Imidazolylpropanolverbindungen nach Anspruch 1, welches

(1) die Umsetzung eines racemischen Epoxids der Formel

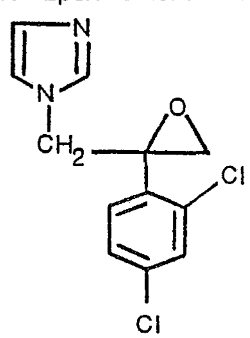

(VIII)

mit einer optisch aktiven Thiolcarbonsäure der Formel A—SH, worin A eine optisch aktive Acylgruppe darstellt, zur Bildung einer Mischung von zwei diastereomeren Isomeren der Formeln

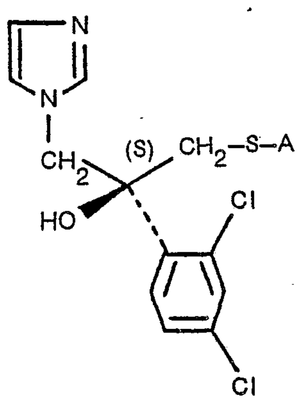

und

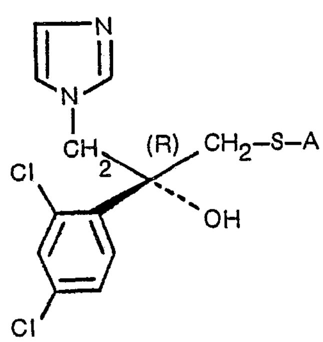

(Xb)

(Xa)

worin A die obige Bedeutung hat;

(2) die Abtrennung des (R)-Isomeren aus dieser Mischung;

(3) die Umsetsung des (R)-Isomeren mit einer Base zur Bildung eines Imidazolylthiols der Formel

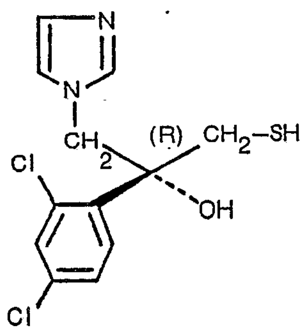

(XI)

und

(4) die Umsetzung des Imidazolylthiols mit einem Alkylierungsmittel der Formel

$$CH_3-(CH_2)_n-X \quad (XII),$$

worin n die obige Bedeutung hat und X für ein Halogenatom, eine Alkylsulfonyloxygruppe oder eine Arylsulfonyloxygruppe steht; umfaßt.

12. Verfahren nach Anspruch 11, worin die optisch aktive Thiolcarbonsäure eine Verbindung der Formel

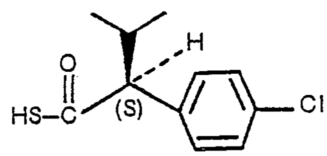

(IX)

ist.

13. Antifungale Zusammensetzung, welche eine anti-fungal wirksame Menge von wenigstens einer der optisch aktiven Imidazolylpropanolverbindungen und ihren Säureadditionssalzen nach Anspruch 1 als ein aktives Ingredienz und einen pharmazeutisch akzeptablen Träger oder ein Verdünnungsmittel umfaßt.

**Patentansprüche für den Vertragsstaat: AT**

1. Verfahren zur Herstellung optisch aktiver Imidazolylpropanolverbindungen der Formel:

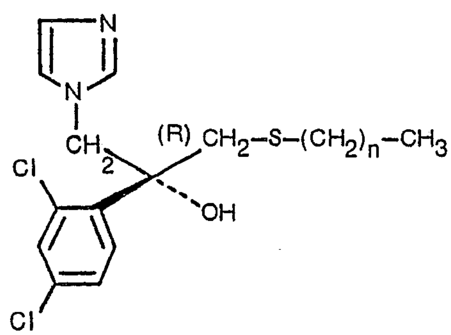

(I)

worin n eine ganze Zahl von 3 oder 4 ist, und ihrer Säureadditionssalze, welches dadurch gekennzeichnet ist, daß ein Epoxid der Formel

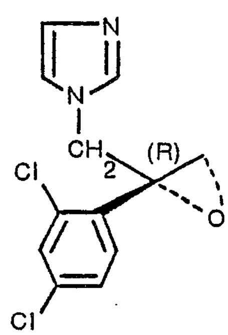

(VI)

mit einer Thiolverbindung der Formel

$$HS—(CH_2)_n—CH_3 \quad (VII),$$

worin n wie oben definiert ist, welche in metallisierter Form vorliegt, umgesetzt wird.

2. Verfahren zur Herstellung optisch aktiver Imidazolylpropanolverbindungen nach Anspruch 1, dadurch gekennzeichnet, daß

(1) ein racemischen Diol der Formel

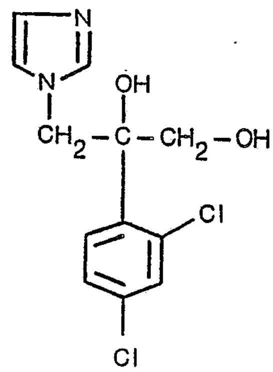

(II)

mit einem optisch aktiven Carbonsäurechlorid der Formel ACl, worin A eine optisch aktive Acylgruppe darstellt, zur Bildung einer Mischung von zwei diastereomeren Isomeren der Formeln

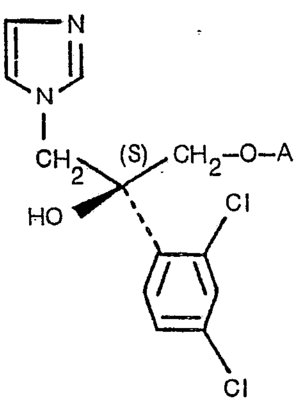

(IVa)

und

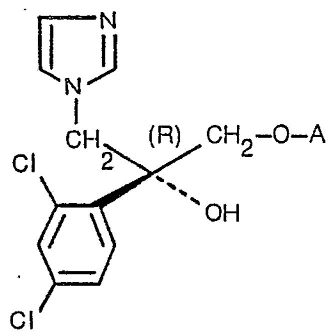

(IVb)

worin A die oben definiert ist, umgesetzt wird;

(2) das (R)-Isomere aus dieser Mischung abgetrennt wird;

(3) das (R)-Isomere zur Bildung eines Diols der Formel

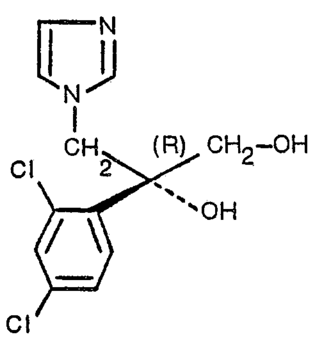

(V)

hydrolysiert wird;

(4) das Diol mit Methansulfonylchlorid umgesetzt wird, gefolgt von einer Behandlung mit einer Base zur Bildung eines Epoxids der Formel

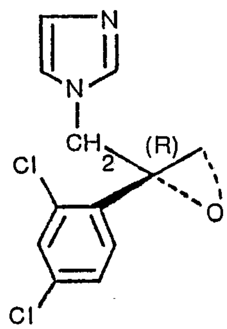

(VI)

und

(5) das Epoxid (VI) mit einer Thiolverbindung der Formel

$$HS—(CH_2)_n—CH_3 \quad (VII),$$

worin n wie oben definiert ist, welche in metallisierter Form vorliegt, umgesetzt wird.

3. Verfahren nach Anspruch 2, dadurch gekennzeichnet, daß das optisch aktive Carbonsäurechlorid (S)-2-(4-Chlorphenyl)-isovalerylchlorid, (+)-α-Methoxy-α-trifluormethylphenylacetylchlorid oder L-Menthoxyacetylchlorid ist.

4. Verfahren zur Herstellung optisch aktiver Imidazolylpropanol verbindungen nach Anspruch 1, dadurch gekennzeichnet, daß eine optisch aktive Imidazolylthiolverbindung der Formel

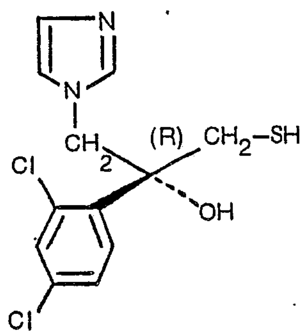

(XI)

mit einem Alkylierungsmittel der Formel

$$CH_3—(CH_2)_n—X \quad (XII),$$

worin n die obige Bedeutung hat und X ein Halogen-atom, eine Alkylsulfonyloxygruppe oder eine Arylsulfonyloxygruppe darstellt, umgesetzt wird.

5. Verfahren nach Anspruch 4, dadurchgekennzeichnet, daß die Umsetzung in Gegenwart einer Ease durchgeführt wird.

6. Verfahren zur Herstellung optisch aktiver Imidazolylpropanolverbindungen nach anspruch 1, dadurch gekennzeichnet, daß

(1) ein racemisches Epoxid der Formel

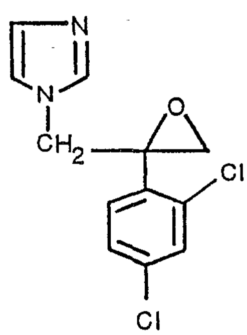

(VIII)

mit einer optisch aktiven Thiolcarbonsäure der Formel A—SH, worin A eine optisch aktive Acylgruppe darstellt, zur Erzielung einer Mischung zweier diastereomerer Isomeren der Formeln

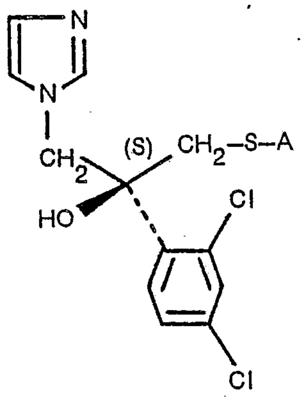

und

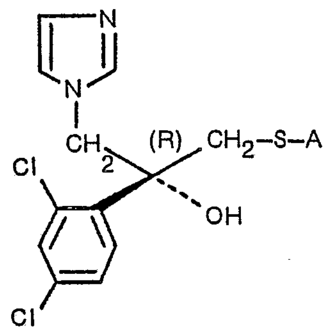

(.Xb)

(Xa)

worin A die oben definiert ist umgesetzt wird;

(2) das (R)-Isomere aus dieser Mischung abgetrennt wird;

(3) das (R)-Isomere mit einer Base zur Bildung eines Imidazolylthiols der Formel

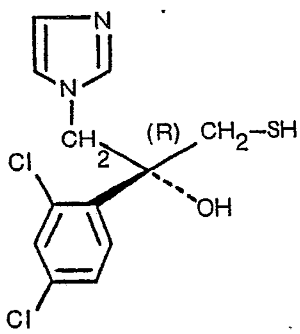

(XI)

umgesetzt wird; und

(4) das Imidazolylthiol mit einem Alkylierungsmittel der Formel

$$CH_3—(CH_2)_n—X \quad (XII),$$

worin n wie oben definiert ist und X für ein Halogenatom, eine Alkylsulfonyloxygruppe oder eine Arylsulfonyloxygruppe steht, umgesetzt wird.

7. Verfahren nach Anspruch 6, dadurch gekennzeichnet, daß als optisch aktive Thiolcarbonsäure eine Verbindung der Formel

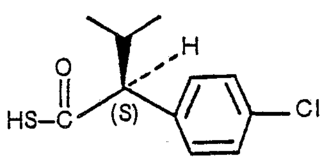

(IX)

eingesetzt wird.

8. Verfahren nach den Ansprüchen 1 bis 7, dadurch gekennzeichnet, daß (R)-3-(n-Butylthio)-2-(2,4-dichlorphenyl)-1-(imidazol-1-yl)-2-propanol hergestellt wird.

9. Verfahren nach den Ansprüchen 1 bis 7, dadurch gekennzeichnet, daß (R)-2-(2,4-Dichlorphenyl)-1-(imidazol-1-yl)-3-(n-pentylthio)-2-propanol hergestellt. wird.

10. Verfahren zur Herstellung einer antifungalen Zusammensetzung, dadurch gekennzeichnet, daß eine antifungal wirksame Menge von wenigstens einer der optisch aktiven Imidazolylpropanolverbindungen und ihrer Säureadditionssalz nach Anspruch 1 als ein aktives Ingredienz und ein pharmazeutisch akzeptabler Träger oder ein Verdünnungsmittel formuliert werden.

**Revendications pour les Etats contractants: BE CH DE FR GB IT LI NL SE**

1. Composé d'imidazolylpropanol optiquement actif répondant à la formule:

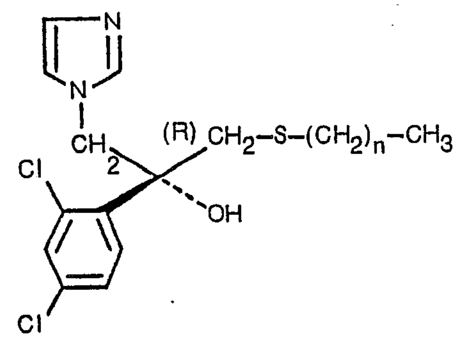

(I)

dans laquelle n est un nombre entier valant 3 ou 4, et ses sels d'addition avec un acide.

2. Composé selon la revendication 1, caractérisé en ce qu'il est le (R)-3-(n-butylthio)-2-(2,4-dichlorophényl)-1-(imidazol-1-yl)-2-propanol.

3. Composé selon la revendication 1, caractérisé en ce qu'il est le (R)-2-(2,4-Dichlorophényl)-1-(imidazol-1-yl)-3-(n-pentylthio)-2-propanol.

4. Procédé de préparation des composé d'imidazolylpropanol optiquement actifs selon la revendication 1, caractérisé en ce qu'il consiste à faire réagir un composé époxy de formule

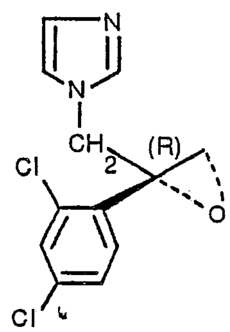

(VI)

avec un thiol de formule:

$$HS-(CH_2)_n-CH_3 \quad (VII)$$

dans laquelle n est tel que défini ci-dessus sous une forme métallisée.

5. Procédé de préparation des composés d'imidazolylpropanol optiquements actifs selon la revendication 1, caractérisé en ce qu'il consiste:

(1) à faire réagir un diol racémique de formule:

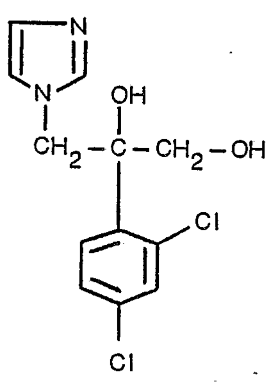

(II)

avec un chlorure d'acide carboxylique optiquement actif de formule: ACl dans laquelle A est un groupe acyle optiquement actif, pour former un mélange de deux isomères diastéréo-isomères de formules:

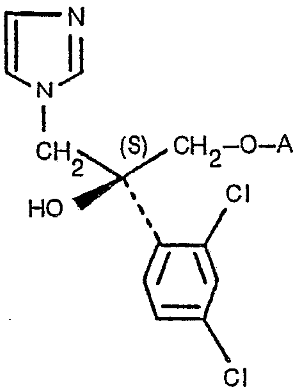

et

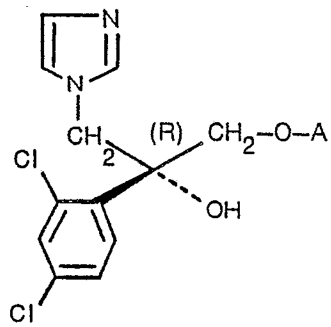

(IVb)

(IVa)

dans laquelle A est tel que défini ci-dessus;

(2) à séparer l'isomère (R) dudit mélange;

(3) à hydrolyzer l'isomère (R) pour donner un diol de formule:

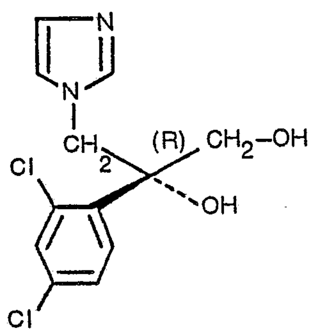

(V)

(4) à faire réagir le diol avec le chlorure de méthanesulfonyle et à poursuivre par un traitement avec une base pour obtenir un composé époxy de formule

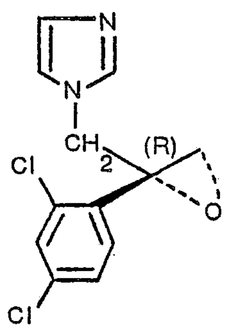

(VI)

et

(5) à faire réagir l'époxy (VI) avec un thiol de formule:

$$HS-(CH_2)_n-CH_3 \quad \text{(VII)}$$

dans laquelle n est tel que défini ci-dessus, sous une forme métallisée.

6. Procédé selon la revendication 5, caractérisé en ce que le chlorure d'acide carboxylique optiquement actif est le chlorure de (S)-2(4-chlorophenyl)isovaléryle, le chlorure de (+)-α-méthoxy-α-trifluorométhylphénylacétyle ou le chlorure de l-menthoxyacétyle.

7. (R)-2-(2,4-dichlorophényl)-1-(imidazol-1-yl)-2,3-propanediol de formule:

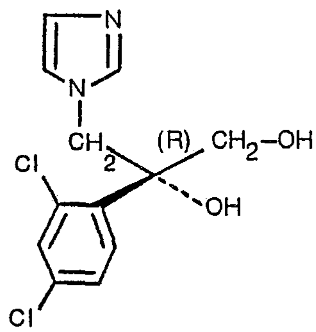

(V)

8. (R)-2-(2,4-dichlorophényl)-1-(imidazol-1-yl)-méthyloxirane de formule:

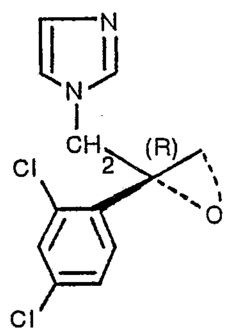

(VI)

9. Procédé de préparation des composés d'imidazolylpropanol optiquement actifs selon la revendication 1, caractérisé en ce qu'il consiste à faire réagir un composé d'imidazolylthiol optiquement actif de formule

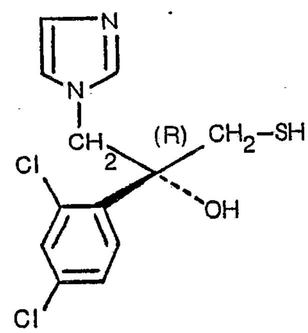

(XI)

avec un agent d'alkylation de formule:

$$CH_3—(CH_2)_n—X. \qquad (XII)$$

dans laquelle n est tel que défini ci-dessus et X est un atome d'halogène, un groupe alkylsulfonyloxy ou un groupe arylsulfonyloxy.

10. Procédé selon la revendication 9, caractérisé en ce que la réaction est effectuée en présence d'une base.

11. Procédé de préparation des composés d'imidazolylpropanol optiquement actifs selon la revendication 1, caractérisé en ce qu'il consiste

(1) à faire réagir un composé époxy racémique de formule:

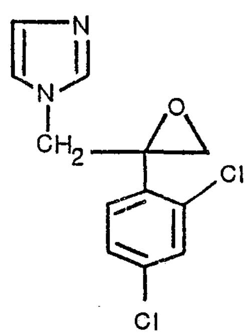

(VIII)

avec un acide thiolocarboxylique optiquement actif de formule: A—SH dans laquelle A est un groupe acyle optiquement actif pour donner un mélange de deux isomères diastéréo-isomères de formules:

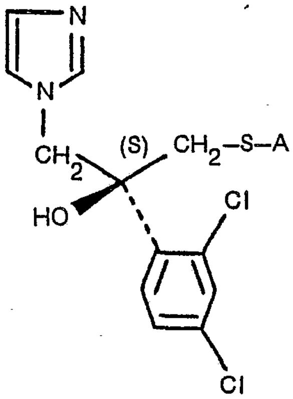

et

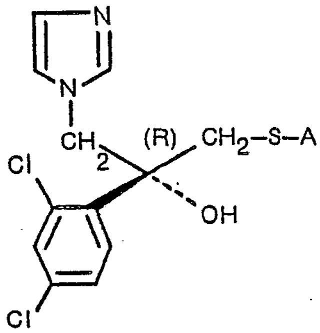

(Xb)

(Xa)

dans lesquelles A est tel que défini ci-dessus;

(2) à séparer l'isomère (R) dudit mélange;

(3) à faire réagir l'isomère (R) avec une base pour obtenir un imidazolylthiol de formule:

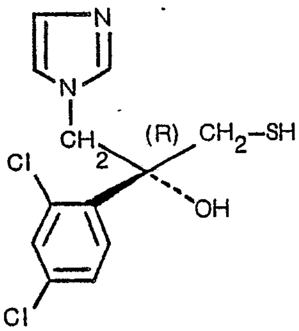

(XI)

et

(4) à faire réagir l'imidazolylthiol avec un agent d'alkylation de formule:

$$CH_3—(CH_2)_n—X \quad (XII)$$

dans laquelle n est tel que défini ci-dessus et X est un atome d'halogène, un groupe alkylsulfonyloxy ou un groupe arylsulfonyloxy.

12. Procédé selon la revendication 11, caractérisé en ce que l'acide thiolocarboxylique optiquement actif est un composé de formule:

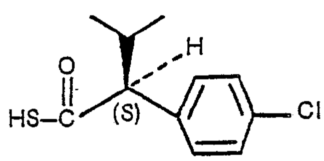

(IX)

13. Composition fongicide caractérisée en ce qu'elle comprend une quantité efficace comme fongicide d'au moins un des composés d'imidazolylpropanol optiquement actifs et de leurs sels d'addition avec un acide selon la revendication 1, à titre d'ingrédient actif et un diluant ou un véhicule pharmaceutiquement acceptable.

**Revendications pour l'Etat contractant: AT**

1. Procédé de préparation des composés d'imidazolylpropanol optiquement actifs répondant à la formulé:

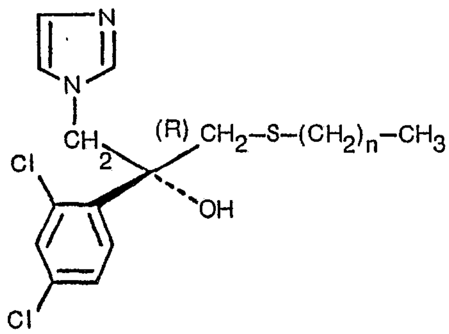

(I)

dans laquelle n est un nombre entier valant 3 ou 4, et de leurs sels d'addition avec un acide, caractérisé en ce qu'il consiste à faire réagir un composé époxy de formule

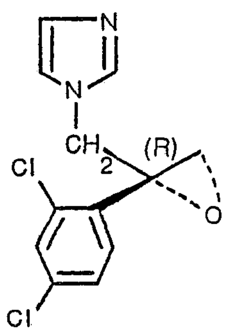

(VI)

avec un thiol de formule:

$$HS—(CH_2)_n—CH_3 \qquad (VII)$$

dans laquelle n est tel que défini ci-dessus, sous une forme métallisée.

2. Procédé de préparation des composés d'imidazolylpropanol optiquement actifs selon la revendication 1, caractérisé en ce qu'il consiste:

(1) à faire réagir un diol racémique de formule:

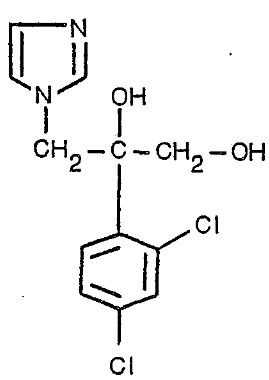

(II)

avec un chlorure d'acide carboxylique optiquement actif de formule: ACl dans laquelle A est un groupe acyle optiquement actif pour former un mélange de deux isomères diastéréo-isomères de formules:

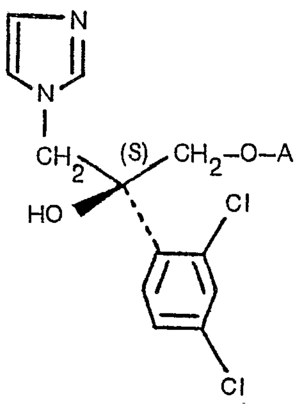

et

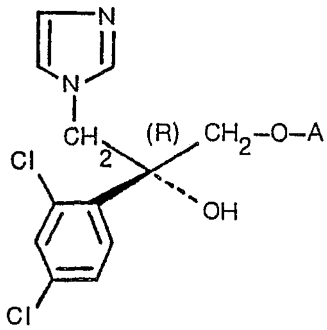

(IVb)

(IVa)

dans laquelles A est tel que défini ci-dessus;

(2) à séparer l'isomère (R) dudit mélange;

(3) à hydrolyzer l'isomère (R) pour obtenir un diol de formule:

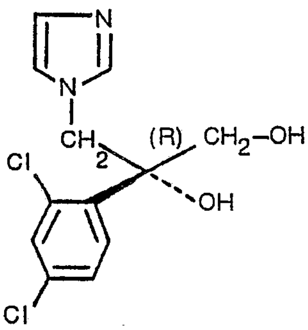

(V)

(4) à faire réagir le diol avec le chlorure de méthanesulfonyle et à poursuivre par un traitement avec une base pour obtenir un composé époxy de formule

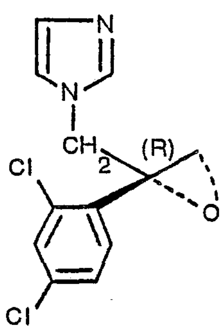

(VI)

et

(5) à faire réagir l'époxy de formule (VI) avec un thiol de formule:

$$HS—(CH_2)_n—CH_3 \quad (VII)$$

dans laquelle n est tel que défini ci-dessus, sous une forme métallisée.

3. Procédé selon la revendication 2 caractérisé en ce que le chlorure d'acide carboxylique optiquement actif est le chlorure de (S)-2-(4-chlorophènyl)isovaléryle, le chlorure de (+)-α-méthoxy-α-trifluorométhylphénylacétyl ou le chlorure de L-menthoxyacétyle.

4. Procédé de préparation des composés d'imidazolylpropanol optiquement actifs selon la revendication 1, caractérisé en ce qu'il consiste à faire réagir un composé d'imidazolylthiol optiquement actif der formule:

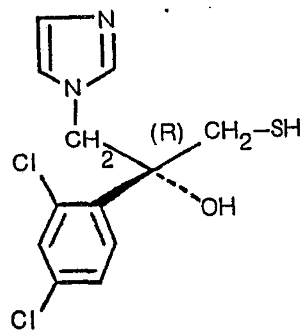

avec un agent d'alkylation de formule:

$$CH_3-(CH_2)_n-X \qquad (XII)$$

dans laquelle n est tel que défini ci-dessus et X est un atome d'halogène, un groupe alkylsulfonyloxy ou un groupe arylsulfonyloxy.

5. Procédé selon la revendication 4, caractérisé en ce que la réaction est effectuée en présence d'une base.

6. Procédé de préparation des composés d'imidazolylpropanol optiquement actifs selon la revendication 1, caractérisé en ce qu'il consiste:

(1) à faire réagir un composé époxy racémique de formule:

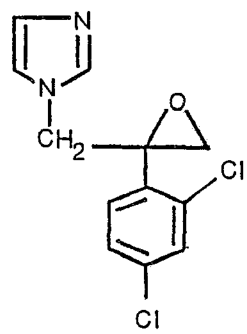

(VIII)

avec un acide thiolocarboxylique optiquement actif de formule: A—SH dans laquelle A est un groupe acyle optiquement actif pour donner un mélange de deux isomères diastéréo-isomères de formules:

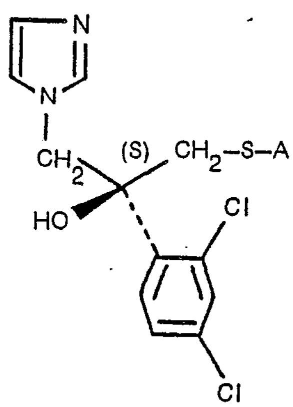

(Xa)

et

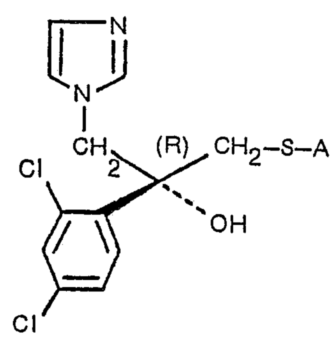

(Xb)

dans lesquelles A est tel que défini ci-dessus;

(2) à séparer l'isomère (R) dudit mélange;

(3) à faire réagir l'isomère (R) avec une base pour obtenir un imidazolylthiol de formule:

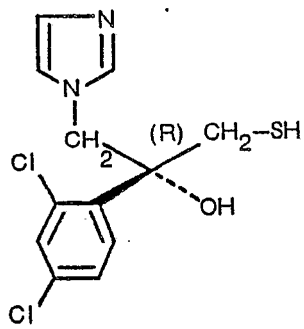

(XI)

et

(4) à faire réagir l'imidazolylthiol avec un agent d'alkylation de formule:

$$CH_3-(CH_2)_n-X \quad (XII)$$

dans laquelle n est tel que défini ci-dessus et X est un atome d'halogène, un groupe alkylsulfonyloxy ou un groupe arylsulfonyloxy.

7. Procédé selon la revendication 6, caractérisé en ce que l'acide thiolocarboxylique optiquement actif est un composé de formule:

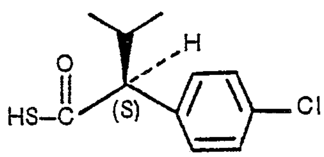

(IX)

8. Procédé selon l'une des revendications 1 à 7 pour la préparation du (R)-3-(n-butylthio)-2-(2,4-dichlorophényl)-1-(imidazol-1-yl)-2-propanol.

9. Procédé selon l'une des revendications 1 à 7 pour la préparation du (R)-2-(2,4-dichlorophényl)-1-(imidazol-1-yl)-3-(n-pentylthio)-2-propanol.

10. Procédé de préparation d'une composition fongicide caractérisé en ce qu'il comprend la fomulation d'une quantité efficace comme fongicide d'au moins un des composés d'imidazolylpropanol optiquement actifs et de leurs sels d'addition avec un acide selon la revendication 1, à titre d'ingrédient actifet d'un diluant ou un véhicule pharmaceutiquement acceptable.

Fig. 1 Antifungal activity in vivo
(Decrease of mortality in Candida-infected mice)